# EUROPEAN PATENT SPECIFICATION

(11) **EP 2 203 426 B1**
(45) Date of publication and mention of the grant of the patent: **31.08.2011**
(21) Application number: 08844327.0
(22) Date of filing: 30.10.2008
(51) Int. Cl.: C07D 213/76, C07D 401/04, A61P 25/00, A61K 31/444

(54) **BENZENESULFONAMIDE COMPOUNDS SUITABLE FOR TREATING DISORDERS THAT RESPOND TO MODULATION OF THE DOPAMINE D3 RECEPTOR**
BENZENSULFONAMID-VERBINDUNGEN ZUR BEHANDLUNG VON ERKRANKUNGEN, DIE AUF DOPAMIN-D3-REZEPTORMODULATION ANSPRECHEN
COMPOSÉS DE BENZÈNESULFONAMIDE APPROPRIÉS POUR LE TRAITEMENT DE TROUBLES QUI RÉPONDENT À UNE MODULATION DU RÉCEPTEUR D<SB>3</SB>DE LA DOPAMINE

(30) Priority: 31.10.2007 EP 07119788
(43) Date of publication of application: 07.07.2010
(73) Proprietor: Abbott GmbH & Co. KG, 65205 Wiesbaden (DE)
(72) Inventor: TURNER, Sean Colm, 67061 Ludwigshafen (DE); HAUPT, Andreas, 67061 Ludwigshafen (DE); BRAJE, Wilfried, 67061 Ludwigshafen (DE); LANGE, Udo, 67061 Ludwigshafen (DE); DRESCHER, Karla, 67061 Ludwigshafen (DE); UNGER, Liliane, 67061 Ludwigshafen (DE); JONGEN-RELO, Ana Lucia, 67061 Ludwigshafen (DE); BESPALOV, Anton, 67061 Ludwigshafen (DE)
(74) Representative: Reitstötter - Kinzebach
(86) International application number: PCT/EP2008/064732
(87) International publication number: WO 2009/056600

(56) References cited:
- WO-A-2004/089905

## Description

### Background Of The Invention

The present invention relates to novel N-(6-piperazin-1-ylpyridin-3-yl)benzenesulfonamide compounds, in particular to the compounds of the formula I as described herein. The compounds possess valuable therapeutic properties and are suitable, in particular, for treating diseases that respond to modulation of the dopamine D₃ receptor.

Neurons obtain their information by way of G protein-coupled receptors, inter alia. A large number of substances exert their effect by way of these receptors. One of them is dopamine. Confirmed findings exist with regard to the presence of dopamine and its physiological function as a neurotransmitter. Disorders in the dopaminergic transmitter system result in diseases of the central nervous system which include, for example, schizophrenia, depression and Parkinson's disease. These diseases, and others, are treated with drugs which interact with the dopamine receptors.

Up until 1990, two subtypes of dopamine receptor had been clearly defined pharmacologically, termed D₁ and D₂ receptors. More recently, a third subtype was found, namely, the D₃ receptor which appears to mediate some effects of antipsychotics and antiparkinsonian drugs (J.C. Schwartz et al., "The Dopamine D3 Receptor as a Target for Antipsychotics" in Novel Antipsychotic Drugs, H.Y. Meltzer, ed., Raven Press, New York 1992, pages 135-144; M. Dooley et al., Drugs and Aging 1998, 12:495-514; J.N. Joyce, Pharmacology and Therapeutics 2001, 90:231-59, "The Dopamine D3 Receptor as a Therapeutic Target for Antipsychotic and Antiparkinsonian Drugs"). Since then, the dopamine receptors have been divided into two families. On the one hand, there is the D₂ group, consisting of D₂, D₃ and D₄ receptors, and, on the other hand, the D₁ group, consisting of D₁ and D₅ receptors.

Whereas D₁ and D₂ receptors are widely distributed, D₃ receptors appear to be expressed regioselectively. Thus, these receptors are preferentially to be found in the limbic system and the projection regions of the mesolimbic dopamine system, especially in the nucleus accumbens, but also in other regions, such as the amygdala. Because of this comparatively regioselective expression, D₃ receptors are regarded as being a target having few side-effects and it is assumed that while a selective D₃ ligand would have the properties of known antipsychotics, it would not have their dopamine D₂ receptor-mediated neurological side-effects (P. Sokoloff et al., Arzneim. Forsch./Drug Res. 42(1):224 (1992), "Localization and Function of the D3 Dopamine Receptor"; P. Sokoloff et al., Nature, 347:146 (1990), "Molecular Cloning and Characterization of a Novel Dopamine Receptor (D3) as a Target for Neuroleptics").

N-(6-Piperazin-1-ylpyridin-3-yl)benzenesulfonamide compounds having an affinity for the dopamine D₃ receptor have been described previously on various occasions, as for example in WO2004/089905. These compounds possess high affinities for the dopamine D₃ receptor, and have therefore been proposed as being suitable for treating diseases of the central nervous system. Unfortunately, their selectivity towards the D₃ receptor is not always satisfactory. Moreover, some of these compounds have an unfavorable DMPK profile (DMPK: metabolic stability and/or pharmacokinetics), and/or might exhibit cardiovascular interactions. Consequently there is an ongoing need to provide new compounds, which have an improved selectivity towards D₃ receptors or an improved pharmacological profile, such as a favorable DMPK profile and/or might exhibit less cardiovaskular interactions.

### Summary Of The Invention

It has now been found that certain N-(6-piperazin-1-ylpyridin-3-yl)benzenesulfonamide compounds exhibit, to a surprising and unexpected degree, highly selective binding to the dopamine D₃ receptor as well as a favorable DMPK profile, in particular in terms of metabolic stability, and/or a favorable cardiovascular profile, i.e. the compounds exhibit less cardiovascular interactions. Such compounds are those having the general formula I, their pharmaceutically tolerable salts and to the N-oxides thereof: 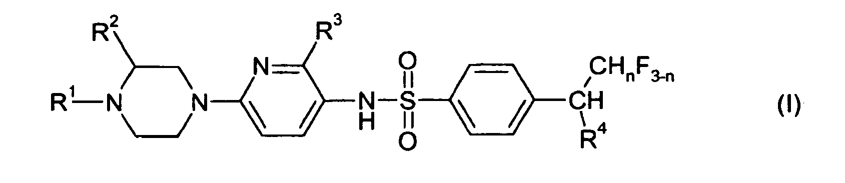 wherein
- R¹: is selected from the group consisting of hydrogen, linear C₁₋c₃ alkyl and fluorinated linear C₁-C₃ alkyl;
- R²: is hydrogen or methyl;
- R³: is selected from the group consisting of hydrogen, halogen, C₁-C₂-alkyl, fluorinated C₁-C₂-alkyl, C₁-C₂-alkoxy and fluorinated C₁-C₂-alkoxy,
- R⁴: is C₁-C₂-alkyl or fluorinated C₁-C₂-alkyl; and
- n: is 0, 1 or 2.

The present invention therefore relates to N-(6-piperazin-1-ylpyridin-3-yl)benzenesulfonamide compounds of the general formula I, as well as to their physiologically tolerated salts and to the N-oxides of the compounds I and of their physiologically tolerated salts.

The present invention also relates to a pharmaceutical composition which comprises at least one N-(6-piperazin-1-ylpyridin-3-yl)benzenesulfonamide compound of the formula I and/or at least one physiologically tolerated salt of I and/or an N-oxide thereof, where appropriate together with physiologically acceptable carriers and/or auxiliary substances.

### Detailed Description Of The Invention

The diseases which respond to the influence of dopamine D₃ receptor antagonists or agonists include disorders and diseases of the central nervous system, in particular affective disturbances, neurotic disturbances, stress disturbances and somatoform disturbances and psychoses, and especially schizophrenia, depression, bipolar disorder, substance abuse (also termed drug abuse), dementia, major depressive disorder, anxiety, autism, attention deficit disorder with or without hyperactivity and personality disorder. In addition, D₃-mediated diseases may include disturbances of kidney function, in particular kidney function disturbances which are caused by diabetes such as diabetes mellitus, also termed as diabetic nephropathy (see WO 00/67847).

According to the invention, one or more compounds of the general formula I having the meanings mentioned at the outset can be used for treating the abovementioned indications. Provided the compounds of the formula I possess one or more centers of asymmetry, it is also possible to use enantiomeric mixtures, in particular racemates, diastereomeric mixtures and tautomeric mixtures; preferred, however, are the respective essentially pure enantiomers, diastereomers and tautomers.

It is likewise possible to use physiologically tolerated salts of the compounds of the formula I, especially acid addition salts with physiologically tolerated acids. Examples of suitable physiologically tolerated organic and inorganic acids are hydrochloric acid, hydrobromic acid, phosphoric acid, nitric acid, sulfuric acid, organic sulfonic acids having from 1 to 12 carbon atoms, e.g. C₁-C₄-alkylsulfonic acids such as methanesulfonic acid, cycloaliphatic sulfonic acids such as S-(+)-10-camphorsulfonic acids and aromatic sulfonic acids such as benzenesulfonic acid and toluenesulfonic acid, di- and tricarboxylic acids and hydroxycarboxylic acids having from 2 to 10 carbon atoms such as oxalic acid, malonic acid, maleic acid, fumaric acid, mucic acid, lactic acid, tartaric acid, citric acid, glycolic acid and adipic acid, as well as cis- and trans-cinnamic acid, furoic acid and benzoic acid. Other utilizable acids are described in Fortschritte der Arzneimittelforschung [Advances in Drug Research], Volume 10, pages 224 ff., Birkhäuser Verlag, Basel and Stuttgart, 1966. The physiologically tolerated salts of compounds of the formula I may be present as the mono-, bis-, tris- and tetrakis-salts, that is, they may contain 1, 2, 3 or 4 of the aforementioned acid molecules per molecule of formula I. The acid molecules may be present in their acidic form or as an anion.

As used herein, C₁-C₃ alkyl is a straight-chain or branched alkyl group having 1, 2 or 3 carbon atoms. Examples of such a group are methyl, ethyl,n-propyl and isopropyl.

As used herein, fluorinated C₁-C₃ alkyl is a straight-chain or branched alkyl group having 1, 2 or 3 carbon atoms, wherein at least one, e.g. 1, 2, 3, 4 or 5 hydrogen atoms or all hydrogen atoms are replaced by fluorine atoms. Examples of such a group are fluoromethyl, difluoromethyl, trifluoromethyl, 2-fluoroethyl, 2,2-difluoroethyl, 2,2,2-trifluoroethyl, 1,1,2,2-tetrafluoroethyl, 3,3,3-trifluoropropyl, 1-methyl-2-fluoroethyl, 1-methyl-2,2-difluoroethyl, 1-methyl-2,2,2-trifluoroethyl and 1, 1, 1, 3, 3, 3-hexafluoropropan-2-yl.

As used herein, C₁-C₂ alkoxy is a straight-chain alkyl group having 1 or 2 carbon atoms which is bound to the remainder of the molecule via an oxygen atom. Examples of such a group are methoxy and ethoxy.

As used herein, fluorinated C₁-C₂ alkoxy is an alkoxy group as defined above, wherein at least one, e.g. 1, 2, 3, 4 or 5 hydrogen atoms are replaced by fluorine atoms. Examples of such a group are fluoromethoxy, difluoromethoxy, trifluoromethoxy, 2-fluoroethoxy, 2,2-difluoroethoxy, 2,2,2-trifluoroethoxy and 1,1,2,2-tetrafluoroethoxy.

A first preferred embodiment of the invention relates to compounds of the formula I, to their pharmacologically tolerated salts and to the N-oxides thereof, wherein R¹ is hydrogen.

Another preferred embodiment of the invention relates to compounds of the formula I, to their pharmacologically tolerated salts and to the N-oxides thereof, wherein R¹ is ethyl or n-propyl.

A further preferred embodiment of the invention relates to compounds of the formula I, wherein R² is methyl. In the compounds, wherein R² is methyl, the carbon atom that carries R² creates a center of chirality. Thus, a specific embodiment of the invention relates to compounds of the formula I, to their pharmacologically tolerated salts and to the N-oxides thereof, wherein R² is methyl and wherein the carbon atom that carries R² has S-configuration. Another specific embodiment of the invention relates to compounds of the formula I, to their pharmacologically tolerated salts and to the N-oxides thereof, wherein R² is methyl and wherein the carbon atom that carries R² has R-configuration.

Likewise preferred are mixtures of compounds wherein the carbon atom that carries R² has S-configuration or R-configuration, respectively. These mixtures may contain equal amounts or non-equal amounts of the compound I, that has R-configuration with regard to the moiety CH-R² and of the compound that has S-configuration with regard to CH-R². Preferred mixtures contain the S-isomer in excess or are enantiomerically pure with regard to CH-R².

The term "enantiomerically pure" means that the mixture contains the respective compound in an enantiomeric excess of at least 80 %, in particular at least 90 % (ee).

A further preferred embodiment of the invention relates to compounds of the formula I, to their pharmacologically tolerated salts and to the N-oxides thereof, wherein R² is hydrogen.

Preference is given to compounds of the formula I, to their pharmacologically tolerated salts and to the N-oxides thereof, wherein R³ is selected from the group consisting of hydrogen, fluorine, chlorine, methyl, fluorinated C₁-alkyl such as trifluoromethyl, methoxy and fluorinated C₁-alkoxy such as difluoromethoxy and trifluoromethoxy. In particular R³ is selected from hydrogen, methyl or methoxy. A particular preferred embodiment of the invention relates to compounds of the formula I, to their pharmacologically tolerated salts and to the N-oxides thereof, wherein R³ is methyl. A further particular preferred embodiment of the invention relates to compounds of the formula I, to their pharmacologically tolerated salts and to the N-oxides thereof, wherein R³ is methoxy.

Preference is given to compounds of the formula I, to their pharmacologically tolerated salts and to the N-oxides thereof, wherein n is 1.

Particular preference is given to compounds of the formula I, to their pharmacologically tolerated salts and to the N-oxides thereof, wherein n is 2.

Preference is given to compounds of the formula I, to their pharmacologically tolerated salts and to the N-oxides thereof, wherein R⁴ is C₁-C₂₋alkyl, in particular methyl. In the compounds of the present invention, the carbon atom that carries R⁴ creates a center of chirality. Thus, a specific embodiment of the invention relates to compounds of the formula I, to their pharmacologically tolerated salts and to the N-oxides thereof, wherein R⁴ is C₁-C₂-alkyl, in particular methyl and wherein the carbon atom that carries R⁴ has S-configuration. Another specific embodiment of the invention relates to compounds of the formula I, to their pharmacologically tolerated salts and to the N-oxides thereof, wherein R⁴ is C₁-C₂-alkyl, in particular methyl and wherein the carbon atom that carries R⁴ has R-configuration.

Likewise preferred are mixtures of compounds wherein the carbon atom that carries R⁴ has S-configuration or R-configuration, respectively. These mixtures may contain equal amounts or non-equal amounts of the compound I, that has R-configuration with regard to the moiety CH-R² and of the compound that has S-configuration with regard to CH-R⁴. Preferred mixtures contain one of the S-isomer in excess or are enantiomerically pure with regard to CH-R⁴.

The term "enantiomerically pure" means that the mixture contains the respective compound in an enantiomeric excess of at least 80 %, in particular at least 90 % (ee).

A particular preferred embodiment la of the invention relates to compounds of the formula I, to their pharmacologically tolerated salts and to the N-oxides thereof, wherein
R¹ is hydrogen;
R² is methyl;
R³ is selected from the group consisting of fluorine, chlorine, methyl, fluorinated C₁-alkyl such as trifluoromethyl, methoxy and fluorinated C₁-alkoxy such as difluoromethoxy and trifluoromethoxy and wherein R³ is in particular methyl;
R⁴ is C₁-C₂-alkyl, in particular methyl; and
wherein the variable n is 1.

A further particular preferred embodiment Ib of the invention relates to compounds of the formula I, to their pharmacologically tolerated salts and to the N-oxides thereof, wherein
R¹ is hydrogen;
R² is methyl;
R³ is selected from the group consisting of fluorine, chlorine, methyl, fluorinated C₁-alkyl such as trifluoromethyl, methoxy and fluorinated C₁-alkoxy such as difluoromethoxy and trifluoromethoxy and wherein R³ is in particular methyl;
R⁴ is C₁-C₂-alkyl, in particular methyl; and
wherein the variable n is 2.

A further particular preferred embodiment Ic of the invention relates to compounds of the formula I, to their pharmacologically tolerated salts and to the N-oxides thereof, wherein
R¹ is hydrogen;
R² is methyl;
R³ is selected from the group consisting of fluorine, chlorine, methyl, fluorinated C₁-alkyl such as trifluoromethyl, methoxy and fluorinated C₁-alkoxy such as difluoromethoxy and trifluoromethoxy and wherein R³ is in particular methyl;
R⁴ is C₁-C₂-alkyl, in particular methyl;and wherein the variable n is 0.

The compounds of the embodiments Ia, Ib and Ic have two centers of chirality and thus may exist in four different stereoisomeric forms, namely in the form of
1. the RR-compound, wherein both the carbon atom carrying the radical R⁴ and the carbon atom carrying the radical R² have R-configuration,
2. the SS-compound, wherein both the carbon atom carrying the radical R⁴ and the carbon atom carrying the radical R² have S-configuration,
3. the RS-compound, wherein the carbon atom carrying the radical R⁴ has R-configuration, while the carbon atom carrying the radical R² has S-configuration, and
4. the SR-compound, wherein the carbon atom carrying the radical R⁴ has S-configuration, while the carbon atom carrying the radical R² has R-configuration.

The compounds of the embodiments Ia, Ib and Ic may be present as diastereomeric mixtures, wherein the RR-, SS-, RS- and SR-compounds may be present in equal or non-equal amounts, as enantiomeric (racemic or non-racemic) mixtures, i.e. as a mixture of the RR- compound with the SS-compound or as a mixture of the RS-compound with the SR-compound and also the form of the pure diastereomers. The term "pure diastereomer" means that the respective diastereomer makes up for at least 80 %, and particular at least 90 % of the respective compound I, i.e. other diastereomers are present in amounts less then 20 %, in particular less than 10 %, based on the total amount of compound I.

Examples of compounds of the embodiment la include 4-(2,2-Difluoro-1-methyl-ethyl)-N-[2-methyl-6-(3-methyl-piperazin-1-yl)-pyridin-3-yl]-benzenesulfonamide and the pharmacologically tolerated salts thereof. This compound may be present as pure stereoisomers, namely:
4-((R)-2,2-Difluoro-1-methyl-ethyl)-N-[2-methyl-6-((R)-3-methyl-piperazin-1-yl)-pyridin-3-yl]-benzenesulfonamide
4-((S)-2, 2-Difluoro-1-methyl-ethyl)-N-[2-methyl-6-((S)-3-methyl-piperazin-1-yl)-pyridin-3-yl]-benzenesulfonamide,
4-((R)-2,2-Difluoro-1-methyl-ethyl)-N-[2-methyl-6-((S)-3-methyl-piperazin-1-yl)-pyridin-3-yl]-benzenesulfonamide, or
4-((S)-2,2-Difluoro-1-methyl-ethyl)-N-[2-methyl-6-((R)-3-methyl-piperazin-1-yl)-pyridin-3-yl]-benzenesulfonamide.
or as diastereomeric mixtures of the RR-, SS-, RS- and SR-compound or as an enantiomeric (racemic or non-racemic) mixture of the RR- compound with the SS-compound or as a mixture of the RS-compound with the SR-compound.

Examples of compounds of the embodiment Ib include 4-(2-Fluoro-1-methyl-ethyl)-N-[2-methyl-6-(3-methyl-piperazin-1-yl)-pyridin-3-yl]-benzenesulfonamide and the pharmacologically tolerated salts thereof. This compound may be present as pure stereoisomers, namely:
4-((R)-2-Fluoro-1-methyl-ethyl)-N-[2-methyl-6-((R)-3-methyl-piperazin-1-yl)-pyridin-3-yl]-benzenesulfonamide
4-((S)-2-Fluoro-1-methyl-ethyl)-N-[2-methyl-6-((S)-3-methyl-piperazin-1-yl)-pyridin-3-yl]-benzenesulfonamide,
4-((R)-2-Fluoro-1-methyl-ethyl)-N-[2-methyl-6-((S)-3-methyl-piperazin-1-yl)-pyridin-3-yl]-benzenesulfonamide, or
4-((S)-2-Fluoro-1-methyl-ethyl)-N-[2-methyl-6-((R)-3-methyl-piperazin-1-yl)-pyridin-3-yl]-benzenesulfonamide.
or as diastereomeric mixtures of the RR-, SS-, RS- and SR-compound or as an enantiomeric (racemic or non-racemic) mixture of the RR- compound with the SS-compound or as a mixture of the RS-compound with the SR-compound.

Examples of compounds of the embodiment Ic include 4-(2,2,2-Trifluoro-1-methylethyl)-N-[2-methyl-6-(3-methyl-piperazin-1-yl)-pyridin-3-yl]-benzenesulfonamide and the pharmacologically tolerated salts thereof. This compound may be present as pure stereoisomers, namely:
4-((R)-2,2,2-Trifluoro-1-methyl-ethyl)-N-[2-methyl-6-((R)-3-methyl-piperazin-1-yl)-pyridin-3-yl]-benzenesulfonamide
4-((S)-2,2,2-Trifluoro-1-methyl-ethyl)-N-[2-methyl-6-((S)-3-methyl-piperazin-1-yl)-pyridin-3-yl]-benzenesulfonamide,
4-((R)-2,2,2-Trifluoro-1-methyl-ethyl)-N-[2-methyl-6-((S)-3-methyl-piperazin-1-yl)-pyridin-3-yl]-benzenesulfonamide, or
4-((S)-2,2,2-Trifluoro-1-methyl-ethyl)-N-[2-methyl-6-((R)-3-methyl-piperazin-1-yl)-pyridin-3-yl]-benzenesulfonamide.
or as diastereomeric mixtures of the RR-, SS-, RS- and SR-compound or as an enantiomeric (racemic or non-racemic) mixture of the RR- compound with the SS-compound or as a mixture of the RS-compound with the SR-compound.

A further particular preferred embodiment Id of the invention relates to compounds of the formula I, to their pharmacologically tolerated salts and to the N-oxides thereof, wherein
R¹ is hydrogen;
R² is hydrogen;
R³ is selected from the group consisting of fluorine, chlorine, methyl, fluorinated C₁-alkyl such as trifluoromethyl, methoxy and fluorinated C₁-alkoxy such as difluoromethoxy and trifluoromethoxy and wherein R³ is in particular methyl;
R⁴ is C₁-C₂-alkyl, in particular methyl; and
wherein the variable n is 1.

A further particular preferred embodiment Ie of the invention relates to compounds of the formula I, to their pharmacologically tolerated salts and to the N-oxides thereof, wherein
R¹ is hydrogen;
R² is hydrogen;
R³ is selected from the group consisting of fluorine, chlorine, methyl, fluorinated C₁-alkyl such as trifluoromethyl, methoxy and fluorinated C₁-alkoxy such as difluoromethoxy and trifluoromethoxy and wherein R³ is in particular methyl;
R⁴ is C₁-C₂₋alkyl, in particular methyl; and
wherein the variable n is 2.

A further particular preferred embodiment If of the invention relates to compounds of the formula I, to their pharmacologically tolerated salts and to the N-oxides thereof, wherein
R¹ is hydrogen;
R² is hydrogen;
R³ is selected from the group consisting of fluorine, chlorine, methyl, fluorinated C₁-alkyl such as trifluoromethyl, methoxy and fluorinated C₁-alkoxy such as difluoromethoxy and trifluoromethoxy and wherein R³ is in particular methyl;
R⁴ is C₁-C₂-alkyl, in particular methyl; and
wherein the variable n is 0.

The compounds of the embodiments Id, Ie and If may be present as racemic or non-racemic mixtures the R-enantiomer with the S-enantiomer and also the form of the pure enantiomer. The term "pure enantiomer" means that the respective enantiomer makes up for at least 80 %, and particular at least 90 % of the respective compound I, i.e. the other enantiomer is present in amounts less then 20 %, in particular less than 10 %, based on the total amount of compound I.

Examples of compounds of the embodiment Id include 4-(2,2-Difluoro-1-methyl-ethyl)-N-[2-methyl-6-piperazin-1-yl-pyridin-3-yl]-benzenesulfonamide and the pharmacologically tolerated salts thereof. This compound may be present as pure enantiomers, namely:
4-((R)-2,2-Difluoro-1-methyl-ethyl)-N-[2-methyl-6-piperazin-1-yl-pyridin-3-yl]-benzenesulfonamide
4-((S)-2,2-Difluoro-1-methyl-ethyl)-N-[2-methyl-6-piperazin-1-yl-pyridin-3-yi]-benzenesulfonamide
or as mixtures of the R- and S-enantiomer.

Examples of compounds of the embodiment Id further include 4-(2,2-Difluoro-1-methyl-ethyl)-N-(2-methoxy-6-piperazin-1-yl-pyrid in-3-yl)-benzenesulfonamide and the pharmacologically tolerated salts thereof. This compound may be present as pure enantiomers, namely:
4-((R)2,2-Difluoro-1-methyl-ethyl)-N-(2-methoxy-6-piperazin-1-yl-pyrid in-3-yl)-benzenesulfonamide
4-((S)2,2-Difluoro-1-methyl-ethyl)-N-(2-methoxy-6-piperazin-1-yl-pyridin-3-yl)-benzenesulfonamide
or as mixtures of the R- and S-enantiomer.

Examples of compounds of the embodiment Ie include 4-(2-Fluoro-1-methyl-ethyl)-N-[2-methyl-6-piperazin-1-yl-pyridin-3-yl]-benzenesulfonamide and the pharmacologically tolerated salts thereof. This compound may be present as pure enantiomers, namely:
4-((R)-2-Fluoro-1-methyl-ethyl)-N-[2-methyl-6-piperazin-1-yl-pyrid in-3-yl]-benzenesulfonamide
4-((S)-2-Fluoro-1-methyl-ethyl)-N-[2-methyl-6-piperazin-1-yl-pyridin-3-yl]-benzenesulfonamide
or as mixtures of the R- and S-enantiomer.

Examples of compounds of the embodiment Ie further include 4-(2-Fluoro-1-methylethyl)-N-(2-methoxy-6-piperazin-1-yl-pyridin-3-yl)-benzenesulfonamide-benzenesulfonamide and the pharmacologically tolerated salts thereof. This compound may be present as pure enantiomers, namely:
4-((R)-2-Fluoro-1-methyl-ethyl)-N-(2-methoxy-6-piperazin-1-yl-pyridin-3-yl)-benzenesulfonamide
4-((S)-2-Fluoro-1-methyl-ethyl)-N-(2-methoxy-6-piperazin-1-yl-pyrid in-3-yl)-benzenesulfonamide
or as mixtures of the R- and S-enantiomer.

Examples of compounds of the embodiment If include 4-(2,2,2-Trifluoro-1-methylethyl)-N-(2-methyl-6-piperazin-1-yl-pyridin-3-yl)-benzenesulfonamide and the pharmacologically tolerated salts thereof. This compound may be present as pure enantiomers, namely:
4-((R)-2,2,2-Trifluoro-1-methyl-ethyl)-N-(2-methyl-6-piperazin-1-yl-pyridin-3-yl)-benzenesulfonamide
4-((S)-2,2,2-Trifluoro-1-methyl-ethyl)-N-(2-methyl-6-piperazin-1-yl-pyridin-3-yl)-benzenesulfonamide
or as mixtures of the R- and S-enantiomer.

Examples of compounds of the embodiment If further include N-(2-Methoxy-6-piperazin-1-yl-pyridin-3-yl)-4-(2,2,2-trifluoro-1-methyl-ethyl)-benzenesulfonamide and the pharmacologically tolerated salts thereof. This compound may be present as pure enantiomers, namely:
N-(2-Methoxy-6-piperazin-1-yl-pyridin-3-yl)-4-((R)-2,2,2-trifluoro-1-methyl-ethyl)-benzenesulfonamide
N-(2-Methoxy-6-piperazin-1-yl-pyridin-3-yl)-4-((S)-2,2,2-trifluoro-1-methyl-ethyl)-benzenesulfonamide

The compounds I according to the invention are prepared in analogy with methods known from the literature. An important approach to the compounds according to the invention is offered by the reaction of a 2-(piperazin-1-yl)-5-aminopyridine compound II with a benzenesulfonic acid derivative III as depicted in scheme 1.

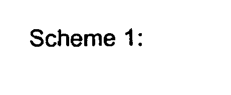

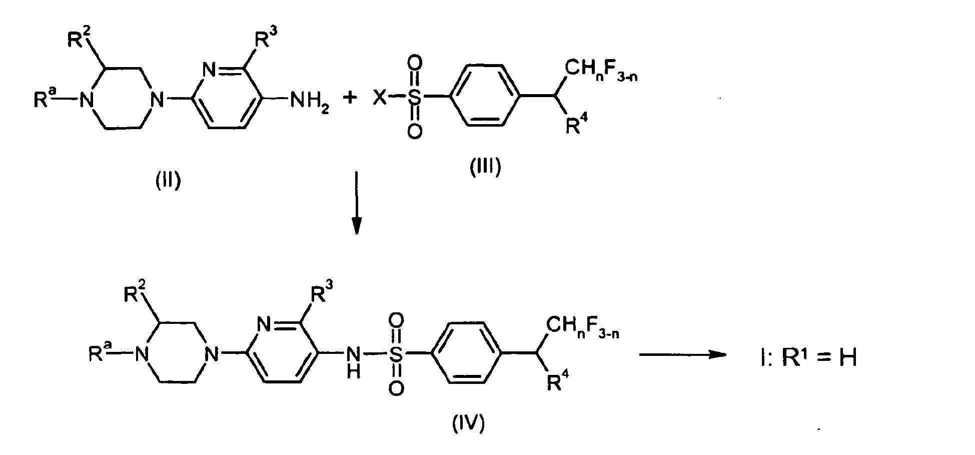

In scheme 1, n, R², R³ and R⁴ have the previously mentioned meanings. R^{a} is a nitrogen protecting group or selected from linear C₁-C₃ alkyl and fluorinated linear C₁-C₃ alkyl. Suitable N-protecting groups are described, for example, in P.J. Kocienski "Protecting Groups", 2nd ed., Georg Thieme Verlag, Stuttgart 2000, pp 186-237 and in the literature cited therein. Preferred examples of N-protecting groups are e.g. oxycarbonyl groups such as C₁-C₆-alkoxycarbonyl, e.g. methoxycarbonyl, ethoxycarbonyl and Boc (tert-butoxycarbonyl) and other oxycarbonyl groups such as benzyloxycarbonyl (Cbz), allyloxycarbonyl, 9-fluorenylmethoxycarbonyl (Fmoc) and 2-trimethylsilylethoxycarbonyl (Teoc), or 2-propenyl (allyl). X is a nucleophilically displaceable leaving group, in particular a halogen atom and, especially, chlorine or bromine.

Compounds of the formula IV, wherein R^{a} is a nitrogen protecting group, in particular a C₁-C₆-alkoxycarbonyl group such as methoxycarbonyl, ethoxycarbonyl and Boc (tert-butoxycarbonyl), are novel and thus form also part of the present invention.

Compounds of the formula IV, wherein R^{a} is linear C₁-C₃ alkyl or fluorinated linear C₁-C₃ alkyl correspond to compounds I, wherein R¹ is linear C₁-C₃ alkyl or fluorinated linear C₁-C₃ alkyl.

The reaction depicted in scheme 1 takes place under the reaction conditions which are customary for preparing arylsulfonamide compounds or arylsulfonic esters, respectively, and which are described, for example, in J. March, Advanced Organic Chemistry, 3rd edition, John Wiley & Sons, New York, 1985, p 444 and the literature cited therein, European J. Org. Chem. 2002 (13), pp. 2094-2108, Tetrahedron 2001, 57 (27) pp. 5885-5895, Bioorganic and Medicinal Chemistry Letters, 2000, 10(8), pp. 835-838 and Synthesis 2000 (1), pp. 103-108.

The reaction customarily takes place in an inert solvent, for example in an ether, such as diethyl ether, diisopropyl ether, methyl tert-butyl ether or tetrahydrofuran, a halohydrocarbon, such as dichloromethane, an aliphatic or cycloaliphatic hydrocarbon, such as pentane, hexane or cyclohexane, or an aromatic hydrocarbon, such as toluene, xylene, cumene and the like, or in a mixture of the abovementioned solvents.

The reaction of compound II with compound III is customarily carried out in the presence of an auxiliary base. Suitable bases are inorganic bases, such as sodium carbonate or potassium carbonate, or sodium hydrogen carbonate or potassium hydrogen carbonate, and organic bases, for example trialkylamines, such as triethylamine, or pyridine compounds, such as pyridine, lutidine and the like. The latter compounds can at the same time serve as solvents. The auxiliary base is customarily employed in at least equimolar quantities, based on the amine compound II.

The reaction of compound II with compound III yields compound IV which, in case R^{a} is an N-protecting group, is deprotected to yield the compound of the general formula I, wherein R¹ is hydrogen. Deprotection of the compound IV can be achieved by standard methods, e.g. by the methods as described in P.J. Kocienski "Protecting Groups", 2nd ed., Georg Thieme Verlag, Stuttgart 2000, pp 186-237 and in the literature cited therein. Customary methods can then be used to react these compounds with an alkylating agent R^{1'}-Z, in which R^{1'} is C₁-C₃-alkyl or fluorinated C₁-C₃-alkyl and Z is a nucleophilically displaceable leaving group (e.g. halogen, such as chlorine, bromine or iodine), resulting in a compound I in which R¹ is C₁-C₃-alkyl or fluorinated C₁-C₃-alkyl. The reaction conditions which are required for this are disclosed, for example, in WO 02/83652, Tetrahedron 2000, 56(38) pp. 7553-7560 and Synlett. 2000 (4), pp. 475-480.

The compounds of the general formula II are known per se or can be prepared in the manner shown in scheme 2.

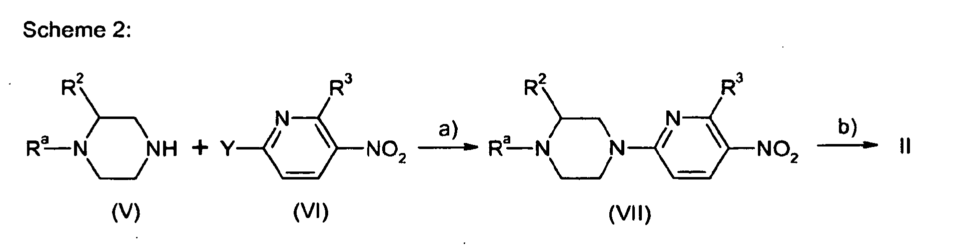

In scheme 2, R^{a}, R², and R³ have the previously mentioned meanings. Y is a nucleophilically displaceable leaving group, in particular a halogen atom, e.g. chlorine or bromine, or an alkylsulfonyl group, e.g. methylsulfonyl.

The reaction depicted in step a) of scheme 2 takes place under the reaction conditions which are customary for a nucleophilic substitution on an aromatic radical and which are described, for example, in Tetrahedron 1999, 55(33), pp. 10243-10252, J. Med. Chem. 1997, 40(22), pp. 3679-3686 and Synthetic Communications, 1993, 23(5), pp. 591-599. Where appropriate, it can be advantageous to convert a ring nitrogen atom in the pyridine ring into its N-oxide (see, for example, Angew. Chem. Int. Ed. Engl., 2002 41 (11), pp. 1937-1940, J. Med. Chem. 1985, 28(2). pp. 248-252 and Tetrahedron Lett. 2002 43(17) pp. 3121-3123). In connection with the subsequent reduction of the nitro group in VII (step b), the N-oxide group is also reduced. For this, the reduction is carried out, for example, in the presence of indium salts.

If Y in the compound VI is bromine, the coupling in step a) of scheme 2, may also be achieved under palladium catalysis in the presence of an auxiliary base, for example an alkali metal carbonate such as cesium carbonate. Particularly suitable palladium catalysts in this connection are palladium(0) compounds or palladium compounds which are able to form a palladium(0) compound under reaction conditions, e.g. palladium dichloride, tetrakis(triphenylphosphine)palladium(0), tris(dibenzylideneacetone)dipalladium(0), advantageously in combination with phosphine ligands, e.g. triarylphosphines, such as triphenylphosphine, trialkylphosphines, such as tributylphosphine, and cycloalkylphosphines, such as tricyclohexylphosphine, and, especially, using phosphine chelate ligands, such as 2,2'-bis(diphenylphosphino)-1,1'-binaphthyf. The conditions which are required for reactions of this nature are described, for example, in Tetrahedron Lett. 2001, 42(22), p. 3681 and Tetrahedron Lett. 2002, 43(12), pp. 2171-2173.

In step b), the nitro group in compound VII is reduced to the NH₂ group to yield compound II. The reaction conditions which are required for step b) correspond to the customary conditions for reducing aromatic nitro groups which have been described extensively in the literature (see, for example, J. March, Advanced Organic Chemistry, 3rd ed., J. Wiley & Sons, New-York, 1985, p. 1183 and the literature cited in this reference). The reduction can be achieved, for example, by reacting the nitro compound VII with a metal such as iron, zinc or tin under acidic reaction conditions, i.e. using nascent hydrogen, or using a complex hydride such as lithium aluminum hydride or sodium borohydride, preferably in the presence of transition metal compounds of nickel or cobalt such as NiCl₂(P(phenyl)₃)₂, or CoCl₂,(see Ono et al. Chem. Ind. (London), 1983 p.480), or using NaBH₂S₃ (see Lalancette et al. Can. J. Chem. 49, 1971, p. 2990), with it being possible to carry out these reductions, depending on the given reagent, in substance or in a solvent or diluent. Alternatively, the reduction of VII to II can be carried out with hydrogen in the presence of a transition metal catalyst, e.g. using hydrogen in the presence of catalysts based on platinum, palladium, nickel, ruthenium or rhodium. The catalysts can contain the transition metal in elemental form or in the form of a complex compound, of a salt or of an oxide of the transition metal, with it being possible, for the purpose of modifying the activity, to use customary coligands, e.g. organic phosphine compounds, such as triphenylphosphine, tricyclohexylphosphine or tri-n-butylphosphines or phosphites. The catalyst is customarily employed in quantities of from 0.001 to 1 mol per mol of compound VII, calculated as catalyst metal. In a preferred variant, the reduction is effected using tin(II) chloride in analogy with the methods described in Bioorganic and Medicinal Chemistry Letters, 2002, 12(15), pp. 1917-1919 and J. Med. Chem. 2002, 45(21), pp. 4679-4688. The reaction of VII with tin(II) chloride is preferably carried out in an inert organic solvent, preferably an alcohol such as methanol, ethanol, isopropanol or butanol.

The N-oxides of compounds of formula I can be obtained by treating a compound of the formula I with an oxidizing agent, in particular an inorganic or organic peroxide or hydroperoxide, such as hydrogen peroxide, or percarboxylic acids, such as peracetic acid, perbenzoic acid or m-chloroperbenzoic acid.

If not otherwise indicated, the above-described reactions are generally carried out in a solvent at temperatures between room temperature and the boiling temperature of the solvent employed. Alternatively, the activation energy which is required for the reaction can be introduced into the reaction mixture using microwaves, something which has proved to be of value, in particular, in the case of the reactions catalyzed by transition metals (with regard to reactions using microwaves, see Tetrahedron 2001, 57, p. 9199 ff. p. 9225 ff. and also, in a general manner, "Microwaves in Organic Synthesis", André Loupy (Ed.), Wiley-VCH 2002).

Examples of solvents which can be used are ethers such as diethyl ether, diisopropyl ether, methyl tert-butyl ether or tetrahydrofuran, aprotic polar solvents such as dimethylformamide, dimethyl sulfoxide, dimethoxyethane and acetonitrile, aromatic hydrocarbons such as toluene and xylene, ketones such as acetone or methyl ethyl ketone, halohydrocarbons such as dichloromethane, trichloromethane and dichloroethane, esters such as ethyl acetate and methyl butyrate, carboxylic acids such as acetic acid or propionic acid, and alcohols such as methanol, ethanol, n-propanol, isopropanol and butanol.

If desired, it is possible for a base to be present in order to neutralize protons which are released in the reactions. Suitable bases include inorganic bases such as sodium carbonate, potassium carbonate, sodium hydrogen carbonate or potassium hydrogen carbonate, alkoxides such as sodium methoxide or sodium ethoxide, alkali metal hydrides such as sodium hydride, organometallic compounds such as butyllithium compounds or alkylmagnesium compounds, and organic nitrogen bases such as triethylamine or pyridine. The latter compounds can at the same time serve as solvents.

The crude product is isolated in a customary manner, as for example by filtering, distilling off the solvent or extracting from the reaction mixture, etc. The resulting compounds can be purified in a customary manner, as for example by means of recrystallizing from a solvent, by means of chromatography or by means of converting into an acid addition salt.

The acid addition salts are prepared in a customary manner by mixing the free base with a corresponding acid, where appropriate in solution in an organic solvent as for example a lower alcohol such as methanol, ethanol, n-propanol or isopropanol, an ether such as methyl tert-butyl ether or diisopropyl ether, a ketone such as acetone or methyl ethyl ketone, or an ester such as ethyl acetate. For example, the free base of formula I and suitable amounts of the corresponding acid, such as from 1 to 4 moles per mol of formula I, are dissolved in a suitable solvent, preferably in a lower alcohol such as methanol, ethanol, n-propanol or isopropanol. Heating may be applied to dissolve the solids, if necessary. Solvents, wherein the acid addition salt of I is insoluble (anti-solvents), might be added to precipitate the salt. Suitable anti-solvents comprise C₁-C₄-alkylesters of C₁-C₄-aliphatic acids such as ethyl acetate, aliphatic and cycloaliphatic hydrocarbons such as hexane, cyclohexane, heptane, etc., di-C₁-C₄-alkylethers such as methyl tert-butyl ether or diisopropyl ether. A part or all of the anti-solvent may be added to the hot solution of the salt and the thus obtained solution is cooled; the remainder of the anti-solvent is then added until the concentration of the salt in the mother liquor is as low as approximately 10 mg/l or lower.

The compounds according to the invention of the formula I are surprisingly highly selective dopamine D₃ receptor ligands. Because of their low affinity for other receptors such as D₁ receptors, D₄ receptors, α1-adrenergic and/or α2-adrenergic receptors, muscarinergic receptors, histamine receptors, opiate receptors and, in particular, dopamine D₂ receptors, the compounds can be expected to give rise to fewer side-effects than do the classic neuroleptics, which are D₂ receptor antagonists.

The high affinity of the compounds according to the invention for D₃ receptors is reflected in very low in-vitro Ki values of as a rule less than 60 nM (nmol/l), preferably of less than 30 nM and, in particular of less than 20 nM. The displacement of [¹²⁵I]-iodosulpride can, for example, be used in receptor binding studies for determining binding affinities for D₃ receptors.

The selectivity of the compounds of the invention for the D₂ receptor relative to the D₃ receptor, expressed as Ki(D₂)/Ki(D₃), is as a rule at least 20, preferably at least 40. The displacement of [³H]SCH23390, [¹²⁵I] iodosulpride or [¹²⁵I] spiperone can be used, for example, in carrying out receptor binding studies on D₁, D₂ and D₄ receptors.

Because of their binding profile, the compounds can be used for treating diseases or disorders which respond to dopamine D₃ ligands, that is, they can be expected to be effective for treating those medical disorders or diseases in which exerting an influence on (modulating) the dopamine D₃ receptors leads to an improvement in the clinical picture or to the disease being cured. Examples of these diseases are disorders or diseases of the central nervous system.

Disorders or diseases of the central nervous system are understood as meaning disorders which affect the spinal cord and, in particular, the brain. Within the meaning of the invention, the term "disorders" denotes disturbances and/or anomalies which are as a rule regarded as being pathological conditions or functions and which can manifest themselves in the form of particular signs, symptoms and/or malfunctions. While the treatment according to the invention can be directed toward individual disorders, that is, anomalies or pathological conditions, it is also possible for several anomalies, which may be causatively linked to each other, to be combined into patterns or syndromes which can be treated in accordance with the invention.

The disorders which can be treated in accordance with the invention are, in particular, psychiatric and neurological disturbances. These disturbances include, in particular, organic disturbances, including symptomatic disturbances such as psychoses of the acute exogenous reaction type or attendant psychoses of organic or exogenous cause as for example in association with metabolic disturbances, infections and endocrinopathologies; endogenous psychoses such as schizophrenia and schizotype and delusional disturbances; affective disturbances such as depressions, major depressive disorder, mania and/or manic-depressive conditions; mixed forms of the above-described disturbances; neurotic and somatoform disturbances and also disturbances in association with stress; dissociative disturbances such as loss of consciousness, clouding of consciousness, double consciousness and personality disturbances; autism; disturbances in attention and waking/sleeping behavior such as behavioral disturbances and emotional disturbances whose onset lies in childhood and youth as for example hyperactivity in children, intellectual deficits such as attention disturbances (attention deficit disorders with or without hyperactivity), memory disturbances and cognitive disturbances such as impaired learning and memory (impaired cognitive function), dementia, narcolepsy and sleep disturbances such as restless legs syndrome; development disturbances; anxiety states; delirium; sexual disturbances such as impotence in men; eating disturbances such as anorexia or bulimia; addiction; bipolar disorder; and other unspecified psychiatric disturbances.

The disorders which can be treated in accordance with the invention also include Parkinson's disease and epilepsy and, in particular, the affective disturbances connected thereto.

Also treatable are addictive diseases (substance abuse), that is, psychic disorders and behavioral disturbances which are caused by the abuse of psychotropic substances such as pharmaceuticals or narcotics, and also other addiction behaviors such as addiction to gaming and/or impulse control disorders not elsewhere classified. Examples of addictive substances include opioids such as morphine, heroin and codeine: cocaine; nicotine; alcohol; substances which interact with the GABA chloride channel complex; sedatives, hypnotics and tranquilizers as for example benzodiazepines; LSD; cannabinoids; psychomotor stimulants such as 3,4-methylenedioxy-N-methylamphetamine (ecstasy); amphetamine and amphetamine-like substances such as methylphenidate; and other stimulants including caffeine. Addictive substances which come particularly into consideration are opioids, cocaine, amphetamine or amphetamine-like substances, nicotine and alcohol.

With regard to the treatment of addiction diseases, particular preference is given to those compounds according to the invention of the formula I which themselves do not possess any psychotropic effect. This can also be observed in a test using rats, which, after having been administered compounds which can be used in accordance with the invention, reduce their self administration of psychotropic substances, for example cocaine.

According to another aspect of the present invention, the compounds according to the invention are suitable for treating disorders whose causes can at least partially be attributed to an anomalous activity of dopamine D₃ receptors.

According to another aspect of the present invention, the treatment is directed, in particular, toward those disorders which can be influenced, within the sense of an expedient medicinal treatment, by the binding of preferably exogeneously administered binding partners (ligands) to dopamine D₃ receptors.

The diseases which can be treated with the compounds according to the invention are frequently characterized by progressive development, that is, the above-described conditions change over the course of time; as a rule, the severity increases and conditions may possibly merge into each other or other conditions may appear in addition to those which already exist.

The compounds according to the invention can be used to treat a large number of signs, symptoms and/or malfunctions which are connected with the disorders of the central nervous system and, in particular, the abovementioned conditions. These signs, symptoms and/or malfunctions include, for example, a disturbed relationship to reality, lack of insight and ability to meet customary social norms or the demands made by life, changes in temperament, changes in individual drives, such as hunger, sleep, thirst, etc., and in mood, disturbances in the ability to observe and combine, changes in personality, in particular emotional lability, hallucinations, ego-disturbances, distracted ness, ambivalence, autism, depersonalization and false perceptions, delusional ideas, chanting speech, lack of synkinesia, short-step gait, flexed posture of trunk and limbs, tremor, poverty of facial expression, monotonous speech, depressions, apathy, impeded spontaneity and decisiveness, impoverished association ability, anxiety, nervous agitation, stammering, social phobia, panic disturbances, withdrawal symptoms in association with dependency, maniform syndromes, states of excitation and confusion, dysphoria, dyskinetic syndromes and tic disorders, such as Huntington's chorea and Gilles-de-la-Tourette's syndrome, vertigo syndromes such as peripheral positional, rotational and oscillatory vertigo, melancholia, hysteria, hypochondria and the like.

Within the meaning of the invention, a treatment also includes a preventive treatment (prophylaxis), in particular as relapse prophylaxis or phase prophylaxis, as well as the treatment of acute or chronic signs, symptoms and/or malfunctions. The treatment can be orientated symptomatically, as for example for the suppression of symptoms.

It can be effected over a short period, be orientated over the medium term or can be a long-term treatment, as for example within the context of a maintenance therapy.

Therefore the compounds according to the invention are preferentially suitable for treating diseases of the central nervous system, in particular for treating affective disorders; neurotic disturbances, stress disturbances and somatoform disturbances and psychoses, and, in particular, for treating schizophrenia and bipolar disorder.

Because of their high selectivity with regard to the D₃ receptor, the compounds I according to the invention are also suitable for treating disturbances of kidney function, in particular disturbances of kidney function which are caused by diabetes (see WO 00/67847) and, especially, diabetic nephropathy.

In addition, compounds of the present invention may possess other pharmacological and /or toxicological properties that render them especially suitable for development as pharmaceuticals. As an example, compounds of formula I having a low affinity for the HERG receptor could be expected to have a reduced likelihood of inducing QT-prolongation (regarded as a one predictor of risk of causing cardiac arrythmia. (For a discussion of QT-prolongation see for example A. Cavalli et al., J. Med. Chem. 2002, 45:3844-3853 and the literature cited therein; a HERG assay is commercially available from GENION Forschungsgesellschaft mbH, Hamburg, Germany).

Within the context of the treatment, the use according to the invention of the described compounds involves a method. In this method, an effective quantity of one or more compounds, as a rule formulated in accordance with pharmaceutical and veterinary practice, is administered to the individual to be treated, preferably a mammal, in particular a human being, productive animal or domestic animal. Whether such a treatment is indicated, and in which form it is to take place, depends on the individual case and is subject to medical assessment (diagnosis) which takes into consideration signs, symptoms and/or malfunctions which are present, the risks of developing particular signs, symptoms and/or malfunctions, and other factors.

As a rule, the treatment is effected by means of single or repeated daily administration, where appropriate together, or alternating, with other active compounds or active compound-containing preparations such that a daily dose of preferably from about 0.01 to 1000 mg/kg, more preferably from 0.1 to 1000 mg/kg of bodyweight in the case of oral administration, or of from about 0.01 to 100 mg/kg, more preferably from 0.1 to 100 mg/kg of bodyweight in the case of parenteral administration, is supplied to an individual to be treated.

The invention also relates to the production of pharmaceutical compositions for treating an individual, preferably a mammal and in particular a human being, a farm animal or a domestic animal. Thus, the compounds are customarily administered in the form of pharmaceutical compositions which comprise a pharmaceutically acceptable excipient together with at least one compound according to the invention and, where appropriate, other active compounds. These compositions can, for example, be administered orally, rectally, vaginally, transdermally, subcutaneously, intravenously, intramuscularly or intranasally.

Examples of suitable pharmaceutical formulations are solid medicinal forms such as powders, granules, tablets (in particular film tablets), lozenges, sachets, cachets, sugar-coated tablets, capsules such as hard gelatin capsules and soft gelatin capsules; suppositories or vaginal medicinal forms; semisolid medicinal forms such as ointments, creams, hydrogels, pastes or plasters; and also liquid medicinal forms such as solutions, emulsions (in particular oil-in-water emulsions), suspensions such as lotions, injection preparations and infusion preparations, and eyedrops and eardrops. Implanted release devices can also be used for administering inhibitors according to the invention. In addition, it is also possible to use liposomes or microspheres.

When producing the compositions, the compounds according to the invention are usually mixed or diluted with an excipient. Excipients can be solid, semisolid or liquid materials which serve as vehicles, carriers or medium for the active compound.

Suitable excipients are listed in the specialist medicinal monographs. In addition, the formulations can comprise pharmaceutically acceptable carriers or customary auxiliary substances, such as glidants; wetting agents; emulsifying and suspending agents; preservatives; antioxidants; antiirritants; chelating agents; coating auxiliaries; emulsion stabilizers; film formers; gel formers; odor masking agents; taste corrigents; resin; hydrocolloids; solvents; solubilizers; neutralizing agents; diffusion accelerators; pigments; quaternary ammonium compounds; refatting and overfatting agents; raw materials for ointments, creams or oils; silicone derivatives; spreading auxiliaries; stabilizers; sterilants; suppository bases; tablet auxiliaries, such as binders, fillers, glidants, disintegrants or coatings; propellants; drying agents; opacifiers; thickeners; waxes; plasticizers and white mineral oils. A formulation in this regard is based on specialist knowledge as described, for example, in Fiedler, H.P., Lexikon der Hilfsstoffe für Pharmazie, Kosmetik und angrenzende Gebiete [Encyclopedia of auxiliary substances for pharmacy, cosmetics and related fields], 4th edition, Aulendorf: ECV-Editio-Kantor-Verlag, 1996.

The following examples serve to explain the invention without limiting it.

The compounds were either characterized via proton-NMR in d₆-dimethylsulfoxid or d-chloroform on a 400 MHz or 500 MHz NMR instrument (Bruker AVANCE), or by mass spectrometry, generally recorded via HPLC-MS in a fast gradient on C18-material (electrospray-ionisation (ESI) mode), or melting point.

The magnetic nuclear resonance spectral properties (NMR) refer to the chemical shifts (δ) expressed in parts per million (ppm). The relative area of the shifts in the ¹H NMR spectrum corresponds to the number of hydrogen atoms for a particular functional type in the molecule. The nature of the shift, as regards multiplicity, is indicated as singlet (s), broad singlet (s. br.), doublet (d), broad doublet (d br.), triplet (t), broad triplet (t br.), quartet (q), quintet (quint.) and multiplet (m).

### Preparation Examples:

### I. Preparation of intermediate compounds IV

Preparation Example 1: (R)-4-{5-[4-((S)-2-Fluoro-1-methyl-ethyl)-benzenesulfonylamino]-6-methyl-pyridin-2-yl}-2-methyl-piperazine-1-carboxylic acid tert-butyl ester

### 1.1 (R)-2-Methyl-4-(6-methyl-5-nitro-pyridin-2-yl)-piperazine

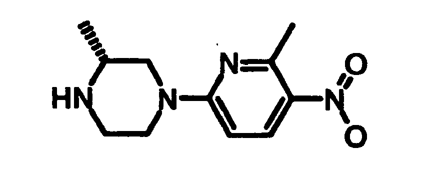

0.72 g of (R)-2-methyl-piperazine (4.17 mmol) were dissolved in 10 mL of N,N-dimethylformamide. 1.165 g of potassium carbonate (8.43 mmol) and 0.44 g of 6-chloro-2-methyl-3-nitro-pyridine were added and the mixture was stirred for 16 h at room temperature. The solvent was evaporated under reduced pressure and the residue was partitioned between water and diethyl ether. The aqueous layer was extracted with diethyl ether and the combined organic phases washed with water, dried over sodium sulfate, filtered and the filtrate was evaporated to dryness to yield 0.85 g of the titel compound.

ESI-MS: 237.1 [M+H]+

### 1.2 (R)-2-Methyl-4-(6-methyl-5-nitro-pyridin-2-yl)-piperazine-1-carboxylic acid tert-butyl ester

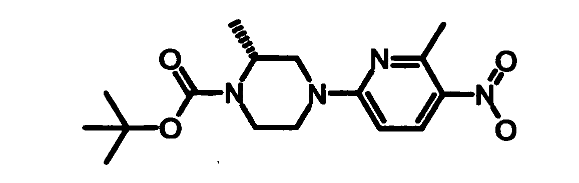

0.85 g of (R)-2-methyl-4-(6-methyl-5-nitro-pyridin-2-yl)-piperazine (3.6 mmol) were dissolved in 12 mL of tetrahydrofuran. A solution of 0.775 g of di-tert.-butyldicarbonate (0.355 mmol) in 3 mL of tetrahydrofuran and 1.5 mL of triethylamine were added. The mixture was stirred for 3 h at room temperature, the solvent was evaporated and the residue was redissolved in diethylether and the solution was washed twice with diluted aqueous ammonium chloride solution. The organic phase was dried over sodium sulfate, filtered and the filtrate evaporated to dryness to yield 1.2 g of the title product.

ESI-MS: 337.1 [M+H]+

### 1.3 (R)-2-Methyl-4-(6-methyl-5-amino-pyridin-2-yl)-piperazine-1-carboxylic acid tert-butyl ester

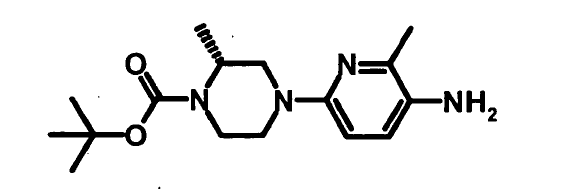

1.2 g of (R)-2-methyl-4-(6-methyl-5-nitro-pyridin-2-yl)-piperazine-1-carboxylic acid tert-butyl ester (3.57 mmol) were dissolved in 20 mL of ethyl acetate. 10% Pd on charcoal and 3 mL of acetic acid were added, and the mixture was hydrogenated for 4 h at 50°C upon completion. The catalyst was filtered off and the filtrate was evaporated to dryness. The residue was treated with water and the pH was adjusted to pH 8-9 with 1 N aqueous sodium hydroxide solution. The aqueous phase was extracted twice with dichloromethane, the organic extracts were dried over sodium sulfate, filtered and the filtrate was evaporated to dryness. The crude product was purified via silica gel chromatography using an ISCO Companion™ instrument (dichloromethane-ethyl acetate 0-50%) to obtain 0.88 g of the title product.

ESI-MS: 307.2 [M+H]+

### 1.4 (R)-4-(5-[4-((S)-2-Fluoro-1-methyl-ethyl)-benzenesulfonylamino]-6-methyl-pyridin-2-yl)-2-methyl-piperazine-1-carboxylic acid tert-butyl ester

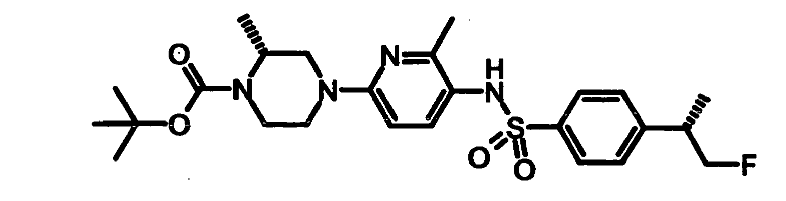

0.105 g of (R)-2-methyl-4-(6-methyl-5-amino-pyridin-2-yl)-piperazine-1-carboxylic acid tert-butyl ester (0.34 mmol) and 0.085 g of ((S)-2-fluoro-1-methylethyl)-benzenesulfonylchloride (0.36 mmol) were dissolved in 5 mL of pyridine and the solution was stirred for 1 h at room temperature. The solvent was evaporated under reduced pressure, the residue was treated with diethyl ether. The organic phase was subsequently washed twice with diluted aqueous ammonium chloride and water. The organic phase was dried over sodium sulfate, filtered, and the solvent was evaporated under reduced pressure. The crude product was purified via silica gel chromatography using an ISCO Companion chromatography system (cyclohexane-ethyl acetate 15-40%) to obtain 0.162 g of product.

ESI-MS: 507.2 [M+H]+

Preparation Example 2: (S)-4{5-[4-((S)-2-Fluoro-1-methyl-ethyl)-benzenesulfonylamino]-6-methyl-pyridin-2-yl}-2-methyl-piperazine-1-carboxylic acid tert-butyl ester

### 2.1 (S)-2-Methyl-4-(6-methyl-5-nitro-pyridin-2-yl)-piperazine

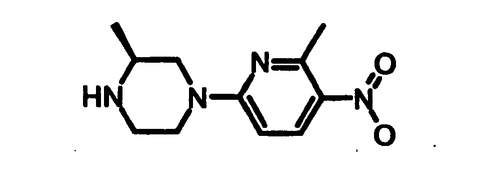

0.72 g of (S)-2-methyl-4-(6-methyl-5-nitro-pyridin-2-yl)-piperazine were prepared as described in preparation example 1.1 for the synthesis of (R)-2-Methyl-4-(6-methyl-5-nitro-pyridin-2-yl)-piperazine.

ESI-MS: 237.1 [M+H]+

¹H-NMR (CDCl₃, 400 Hz): δ [ppm] 8.2 (d, 1H), 6.45 (d, 1H), 4.3-4.45 (m, 2H), 3.1 (m, 1H), 3.0 (m, 1H), 2.7-2.95 (m, 2H), 2.75 (s, 3H), 2.65 (m, 1H), 1.15 (s, 3H).

### 2.2 (S)-2-Methyl-4-(6-methyl-5-nitro-pyridin-2-yl)-piperazine-1-carboxylic acid tert-butyl ester

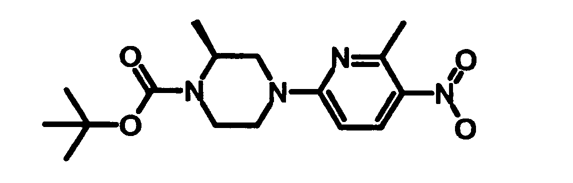

1.13 g of (S)-2-methyl-4-(6-methyl-5-nitro-pyridin-2-yl)-piperazine-1-carboxylic acid tert-butyl ester were prepared as described in preparation example 1.2 for the synthesis of (R)-2-Methyl-4-(6-methyl-5-nitro-pyridin-2-yl)-piperazine-1-carboxylic acid tert-butyl ester.

ESI-MS: 337.2 [M+H]+

### 2.3 (S)-2-Methyl-4-(6-methyl-5-amino-pyridin-2-yl)-piperazine-1-carboxylic acid tert-butyl ester

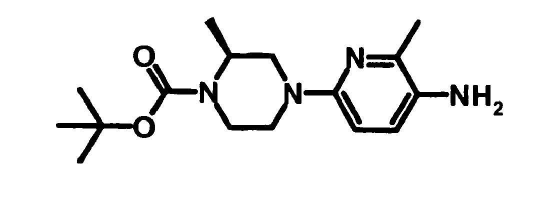

1.13 g of (S)-2-methyl-4-(6-methyl-5-nitro-pyridin-2-yl)-piperazine-1-carboxylic acid tert-butyl ester (3.36 mmol) were dissolved in 65 mL of methanol and 5 mL of ethyl acetate and the solution was subjected to hydrogenation in an H-cube™ (Thalos Ltd.) at 40°C (flow rate 1 mUmin). The solvents were removed under reduced pressure and the residue was purified via silica gel chromatography using an ISCO Companion™ instrument (dichloromethane-ethyl acetate 0-45%) to obtain 0.58 g of the title product.

ESI-MS: 307.2 [M+H]+

### 2.4 (S)-4-{5-[4-((S)-2-Fluoro-1-methyl-ethyl)-benzenesulfonylamino]-6-methyl-pyridin-2-yl}-2-methyl-piperazine-1-carboxylic acid tert-butyl ester

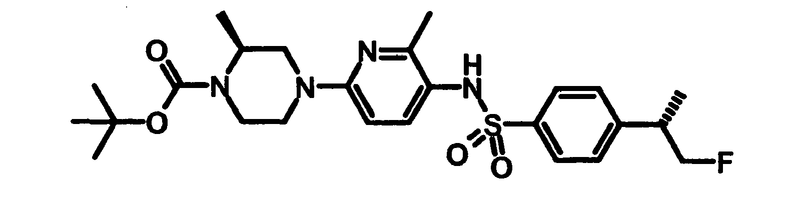

0.105 g of (S)-2-methyl-4-(6-methyl-5-amino-pyridin-2-yl)-piperazine-1-carboxylic acid tert-butyl ester (0.34 mmol) and 0.085 g of ((S)-2-fluoro-1-methylethyl)-benzenesulfonylchloride (0.36 mmol) were dissolved in 5 mL of pyridine and the solution was stirred for 1 h at room temperature. The solvent was evaporated under reduced pressure, the residue was treated with diethyl ether. The organic phase was subsequently washed twice with diluted aqueous ammonium chloride and water. The organic phase was dried over sodium sulfate, filtered, and the solvent was evaporated under reduced pressure. The crude product was purified via silica gel chromatography using an ISCO Companion chromatography system (cyclohexane-ethyl acetate 15-40%) to obtain 0.162 g of product.

ESI-MS: 507.2 [M+H]+

### Preparation Example 3: (S)-4-{5-[4-((R)-2-Fluoro-1-methyl-ethyl)-benzenesulfonylamino]-6-methyl-pyridin-2-yl}-2-methyl-piperazine-1-carboxylic acid tert-butyl ester

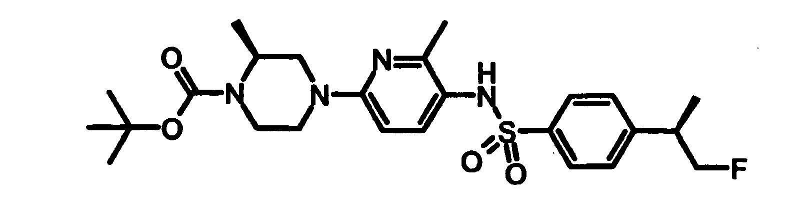

The compound was prepared as described for the synthesis of (S)-4-{5-[4-((S)-2-fluoro-1-methyl-ethyl)-benzenesulfonylamino]-6-methyl-pyridin-2-yl}-2-methylpiperazine-1-carboxylic acid tert-butyl ester in preparation example 2.4 to obtain 0.194 g of product.

ESI-MS: 507.2 [M+H]+

### Preparation Example 4: 4-{5-[4-((S)-2-Fluoro-1-methyl-ethyl)-benzenesulfonylamino]-6-methyl-pyridin-2-yl}-piperazine-1-carboxylic acid tert-butyl ester

### 4.1 4-(6-Methyl-5-amino-pyridin-2-yl)-piperazine-1-carboxylic acid tert-butyl ester

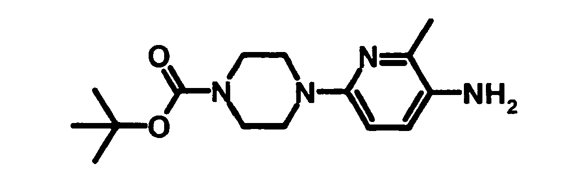

The title compound was prepared by analogy to the methods described in preparation examples 1.1 to 1.3

### 4.2 4-{5-[4-((S)-2-Fluoro-1-methyl-ethyl)-benzenesulfonylamino]-6-methyl-pyridin-2-yl}-piperazine-1-carboxylic acid tert-butyl ester

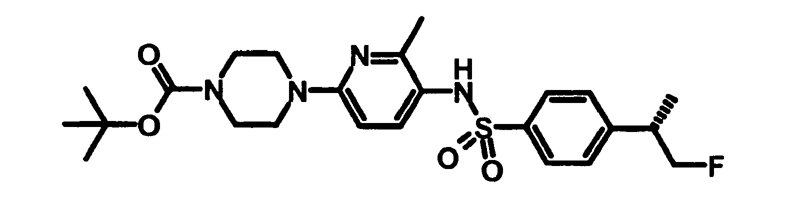

The compound was prepared as described for the synthesis of (S)-4-{5-[4-((S)-2-fluoro-1-methyl-ethyl)-benzenesulfonylamino]-6-methyl-pyridin-2-yl}-2-methylpiperazine-1-carboxylic acid tert-butyl ester in preparation example 2.4.

ESI-MS: 493.2 [M+H]+

### Preparation Example 5: 4-{5-[4-((R)-2-Fluoro-1-methyl-ethyl)-benzenesulfonylamino]-6-methyl-pyridin-2-yl}-piperazine-1-carboxylic acid tert-butyl ester

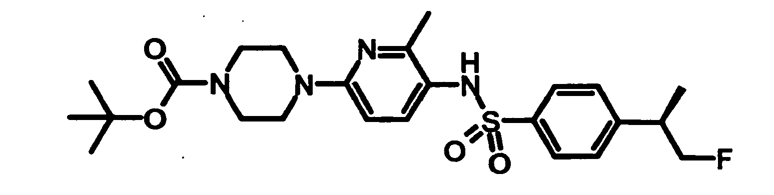

The compound was prepared as described for the synthesis of (S)-4-{5-[4-((S)-2-Fluoro-1-methyl-ethyl)-benzenesulfonylamino]-6-methyl-pyridin-2-yl}-2-methylpiperazine-1-carboxylic acid tert-butyl ester in preparation example 2.4.

ESI-MS: 493.2 [M+H]⁺

### Preparation Example 6: (R)-4-{5-[4-((R)-2-Fluoro-1-methyl-ethyl)-benzenesulfonylamino]-6-methyl-pyridin-2-yl}-2-methyl-piperazine-1-carboxylic acid tert-butyl ester

The compound was prepared as described for the synthesis of (S)-4-{5-[4-((S)-2-Fluoro-1-methyl-ethyl)-benzenesulfonylamino]-6-methyl-pyridin-2-yl}-2-methylpiperazine-1-carboxylic acid tert-butyl ester in preparation example 2.4.

ESI-MS: 507.2 [M+H]+

### Preparation Example 7: 4-{5-[4-(2,2-Difluoro-1-methyl-ethyl)-benzenesulfonylamino]-6-methyl-pyridin-2-yl}-piperazine-1-carboxylic acid tert-butyl ester 450 mg, yield 86%.

### II. Preparation of the compounds I

### Example 1: 4-((S)-2-Fluoro-1-methyl-ethyl)-N-[2-methyl-6-((S)-3-methyl-piperazin-1-yl)-pyridin-3-yl]-benzenesulfonamide

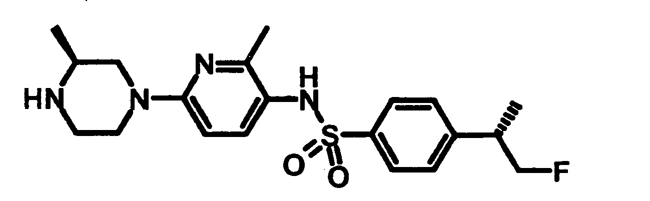

0.162 g of (S)-4-{5-[4-((S)-2-fluoro-1-methyl-ethyl)-benzenesulfonylamino]-6-methyl-pyridin-2-yl}-2-methyl-piperazine-1-carboxylic acid tert-butyl ester (0.32 mmol) were dissolved in 5 mL of dichloromethane. To the solution, 1.5 mL of 6N hydrochloric acid in isopropanol were added. The mixture was stirred for 16 h at room temperature and the solvent was evaporated. The residue was dissolved in water and the solution was adjusted to pH 8-9 with saturated aqueous sodium bicarbonate. The mixture was extracted three times with diethyl ether. The combined organic layers were washed with water, dried over sodium sulfate, filtered, and the solvent was evaporated under reduced pressure. The crude product was treated with 0.3 mL of acetonitrile (containing 0.1 % trifluoroacetic acid) and 0.3 mL acetonitril/water (containing 0.1% trifluoroacetic acid). The formed precipitate was collected, washed with acetonitril/water 1:1 and dried to yield 0.025 g of the title product.

ESI-MS: 407.2 [M+H]⁺

¹H-NMR (CDCl₃, 400 Hz): δ [ppm] 7.7 (d, 2H), 7.4 (d, 2H), 7.3 d, 1H), 6.4 (d, 1H), 4.55 (m, 1H), 4.4, m, 1H), 4.1 (t, 2H), 3.15 (m, 1H), 3.1 (m, 1H), 2.7-2.95 (several m, 3H), 2.4 (t, 1H), 2.0 (s, 3H), 1.3 (d, 3H), 1.1 (d, 3H).

### Example 2: 4-((R)-2-Fluoro-1-methyl-ethyl)-N-[2-methyl-6-((S)-3-methyl-piperazin-1-yl)-pyridin-3-yl]-benzenesulfonamide

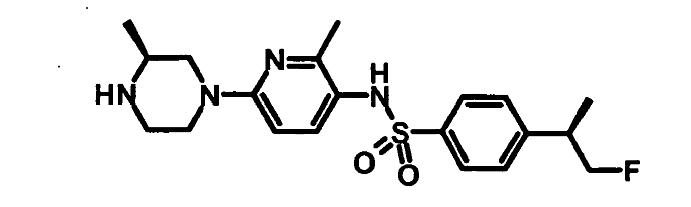

0.194 g (S)-4-{5-[4-((R)-2-fluoro-1-methyl-ethyl)-benzenesulfonylamino]-6-methyl-pyridin-2-yl}-2-methyl-piperazine-1-carboxylic acid tert-butyl ester (0.38 mmol) were dissolved in 5 mL of dichloromethane. To the solution, 1.5 mL of 6N hydrochlorid acid in isopropanol were added. The mixture was stirred for 16 h at room temperature and the solvent was evaporated. The residue was dissolved in water and the solution was adjusted to pH 8-9 with saturated aqueous sodium bicarbonate. The mixture was extracted three times with diethyl ether. The combined organic layers were washed with water, dried over sodium sulfate, filtered, and the solvent was evaporated under reduced pressure to yield 0.128 g of the title product.

ESI-MS: 407.2 [M+H]⁺

¹H-NMR (CDCl₃, 400 Hz): δ [ppm] 7.7 (d, 2H), 7.4 (d, 2H), 7.3 d, 1H), 6.4 (d, 1H), 4.55 (m, 1H), 4.4, m, 1H), 4.1 (t, 2H), 3.15 (m, 1H), 3.1 (m, 1H), 2.7-2.95 (several m, 3H), 2.4 (t, 1H), 2.0 (s, 3H), 1.3 (d, 3H), 1.1 (d, 3H).

If not stated otherwise, the compounds of the following examples 3 to 18 were prepared by analogy to the methods disclosed in Examples 1 and 2.

### Example 3: 4-((R)-2-Fluoro-1-methyl-ethyl)-N-[2-methyl-6-((R)-3-methyl-piperazin-1-yl)-pyridin-3-yl]-benzenesulfonamide

### Example 4: 4-((S)-2-Fluoro-1-methyl-ethyl)-N-[2-methyl-6-((R)-3-methyl-piperazin-1-yl)-pyridin-3-yl]-benzenesulfonamide

### Example 5: 4-((R)-2-Fluoro-1-methyl-ethyl)-N-[2-methyl-6-piperazin-1-yl-pyridin-3-yl]-benzenesulfonamide

100 mg, yield 99%. ESI-MS: 393.1 [M+H]⁺

¹H-NMR (DMSO-d₆, 400 Hz): δ [ppm] 1.24 (d, 3H), 1.98 (s, 3H), 3.16 (m, 4H), 3.23 (m, 1H), 3.67 (m, 4H), 4.48 (d, 1H), 4.60 (d, 1H), 6.66 (d, 1H), 7.12 (d, 1H), 7.50 (d, 2H), 7.60 (d,2H), 9.00 (br s, 1H), 9.43 (s, 1H).

### Example 6: 4-((S)-2-Fluoro-1-methyl-ethyl)-N-[2-methyl-6-piperazin-1-yl-pyridin-3-yl]-benzenesulfonamide

460 mg, yield 99%. ESI-MS: 393.1 [M+H]⁺

¹H-NMR (DMSO-d₆, 400 Hz): δ [ppm] 1.24 (d, 3H), 1.98 (s, 3H), 3.16 (m, 4H), 3.23 (m, 1H), 3.67 (m, 4H), 4.48 (d, 1H), 4.60 (d, 1H), 6.66 (d, 1H), 7.12 (d, 1H), 7.50 (d, 2H), 7.60 (d,2H), 9.00 (br s, 1H), 9.43 (s, 1H).

### Example 7 (comparative): N-(5-Bromo-2-methyl-6-piperazin-1-yl-pyridin-3-yl)-4-((S)-2-fluoro-1-methyl-ethyl)-benzenesulfonamide

4-{5-[4-((S)-2-Fluoro-1-methyl-ethyl)-benzenesulfonylamino]-6-methyl-pyridin-2-yl}-piperazine-1-carboxylic acid tert-butyl ester (370 mg, 0.754 mmol) was dissolved in acetonitrile (3 mL) and N-bromosuccinimide was added (201 mg, 1.13 mmol). The resulting solution was heated at 80°C under microwave irradiation for 35 mins. The cooled solution was then partitioned between NaHCO₃ solution and dichloromethane. The organic layer was separated, dried (MgSO₄), filtered and purified by column chromatography to give the desired product. Yellow solid. Amount 135 mg. Yield 38%. Characterised as HCl salt:

ESI-MS: 473.1, 471.1 [M+H]⁺

¹H-NMR (CDCl₃, 400 Hz): δ [ppm] 1.24 (d, 3H), 2.02 (s, 3H), 3.18 (m, 4H), 3.25 (m, 1H), 3.40 (m, 4H), 4.52 (dd, 2H), 7.39 (s, 1H), 7.54 (d, 2H), 7.67 (d, 2H), 9.15 (br s, 2H), 9.85 (s, 1H).

### Example 8: 4-(2,2-Difluoro-1-methyl-ethyl)-N-(2-methyl-6-piperazin-1-yl-pyridin-3-yl)-benzenesulfonamide

420 mg, yield 99%. ESI-MS: 411.1 [M+H]⁺

¹H-NMR (CD₃OD, 400 Hz): δ [ppm] 1.38 (d, 3H), 2.00 (s, 3H), 3.28 (m, 5H), 3.79 (m, 4H), 5.97 (td, 1H), 6.63 (d, 1H), 7.22 (d, 1H), 7.50 (d, 2H), 7.64 (d, 2H).

### Example 9: 4-((S)-2,2-Difluoro-1-methyl-ethyl)-N-(2-methyl-6-piperazin-1-yl-pyridin-3-yl)-benzenesulfonamide

181 mg, yield 99%. ESI-MS: 411.1 [M+H]⁺

¹H-NMR (CD₃OD, 400 Hz): δ [ppm] 1.38 (d, 3H), 2.00 (s, 3H), 3.28 (m, 5H), 3.79 (m, 4H), 5.97 (td, 1H), 6.63 (d, 1H), 7.22 (d, 1H), 7.50 (d, 2H), 7.64 (d, 2H).

### Example 10: 4-((R)-2,2-Difluoro-1-methyl-ethyl)-N-(2-methyl-6-piperazin-1-yl-pyridin-3-yl)-benzenesulfonamide

230 mg, yield 99%. ESI-MS: 411.1 [M+H]⁺

¹H-NMR (CD₃OD, 400 Hz): δ [ppm] 1.38 (d, 3H), 2.00 (s, 3H), 3.28 (m, 5H), 3.79 (m, 4H), 5.97 (td, 1H), 6.63 (d, 1H), 7.22 (d, 1H), 7.50 (d, 2H), 7.64 (d, 2H).

### Example 11: 4-(2,2,2-Trifluoro-1-methyl-ethyl)-N-(2-methyl-6-piperazin-1-yl-pyridin-3-yl)-benzenesulfonamide

Characterised as HCl salt:

ESI-MS: 430.1 [M+H]+

¹H-NMR (DMSO-d₆, 400 Hz): δ [ppm] 1.45 (d, 3H), 1.90 (s, 3H), 3.14 (m, 4H), 3.67 (m, 4H), 4.98 (m, 1H), 6.68 (d, 1H), 7.12 (d, 1H), 7.63 (d, 4H), 8.93 (s, 2H), 9.55 (s, 1H).

### Example 12: 4-((R)-2,2,2-Trifluoro-1-methyl-ethyl)-N-(2-methyl-6-piperazin-1-yl-pyridin-3-yl)-benzenesulfonamide

420 mg, yield 99%. Characterised as HCl salt:

ESI-MS: 430.1 [M+H]⁺

¹H-NMR (DMSO-d₆, 400 Hz): δ [ppm] 1.45 (d, 3H), 1.90 (s, 3H), 3.14 (m, 4H), 3.67 (m, 4H), 4.98 (m, 1H), 6.68 (d, 1H), 7.12 (d, 1H), 7.63 (d, 4H), 8.93 (s, 2H), 9.55 (s, 1H).

### Example 13: 4-((S)-2,2,2-Trifluoro-1-methyl-ethyl)-N-(2-methyl-6-piperazin-1-yl-pyridin-3-yl)-benzenesulfonamide

62 mg, yield 47%. Characterised as HCl salt:

ESI-MS: 430.1 [M+H]⁺

¹H-NMR (DMSO-d₆, 400 Hz): δ [ppm] 1.45 (d, 3H), 1.90 (s, 3H), 3.14 (m, 4H), 3.67 (m, 4H), 4.98 (m, 1H), 6.68 (d, 1H), 7.12 (d, 1H), 7.63 (d, 4H), 8.93 (s, 2H), 9.55 (s, 1H).

### Example 14: 4-((R)-2-Fluoro-1-methyl-ethyl)-N-(2-methoxy-6-piperazin-1-yl-pyridin-3-yl)-benzenesulfonamide

160mg, yield 80%. Characterised as HCl salt:

ESI-MS: 409.1 [M+H]⁺

¹H-NMR (DMSO-d₆, 400 Hz): δ [ppm] 1.23 (d, 3H), 3.12 (m, 4H), 3.18 (m, 1H), 3.35 (s, 3H), 3.67 (m, 4H), 4.52 (dd, 2H), 6.37 (d, 1H), 7.34 (d, 1H), 7.45 (d, 2H), 7.59 (d, 2H), 9.28 (m, 3H).

### Example 15: 4-((S)-2-Fluoro-1-methyl-ethyl)-N-(2-methoxy-6-piperazin-1-yl-pyridin-3-yl)-benzenesulfonamide

24mg, yield 18%. Characterised as HCl salt:

ESI-MS: 409.1 [M+H]+

¹H-NMR (DMSO-d₆, 400 Hz): δ [ppm] 1.23 (d, 3H), 3.12 (m, 4H), 3.18 (m, 1H), 3.35 (s, 3H), 3.67 (m, 4H), 4.52 (dd, 2H), 6.37 (d, 1H), 7.34 (d, 1H), 7.45 (d, 2H), 7.59 (d, 2H), 9.28 (m, 3H).

### Example 16: 4-(2,2-Difluoro-1-methyl-ethyl)-N-(2-methoxy-6-piperazin-1-yl-pyridin-3-yl)-benzenesulfonamide

57mg, yield 28%. Characterised as HCl salt:

ESI-MS: 427.1 [M+H]⁺

¹H-NMR (CD₃OD, 400 Hz): δ [ppm] 1.38 (d, 3H), 3.28 (m, 4H), 3.42 (s, 3H), 3.48 (m, 1H), 3.74 (m, 4H), 5.97 (dt, 1H), 6.39 (d, 1H), 7.43 (d, 2H), 7.60 (d, 1H), 7.65 (d, 2H).

### Example 17: N-(2-Methoxy-6-piperazin-1-yl-pyridin-3-yl)-4-(2,2,2-trifluoro-1-methylethyl)-benzenesulfonamide

42mg, yield 31 %.

ESI-MS: 445.1 [M+H]⁺

¹H-NMR (CDCl₃, 400 Hz): δ [ppm] 1.48 (d, 3H), 3.10 (m, 4H), 3.38 (s, 3H), 3.43 (m, 1H), 3.59 (m, 4H), 5.20 (br s, 2H), 6.17 (d, 1H), 7.37 (d, 2H), 7.63 (m, 3H).

### Example 18: N-(2-Methoxy-6-piperazin-1-yl-pyridin-3-yl)-4-((S)-2,2,2-trifluoro-1-methylethyl)-benzenesulfonamide

45mg, yield 62%.

ESI-MS: 445.1 [M+H]⁺

¹H-NMR (CDCl₃, 400 Hz): δ [ppm] 1.48 (d, 3H), 3.10 (m, 4H), 3.38 (s, 3H), 3.43 (m, 1H), 3.59 (m, 4H), 5.20 (br s, 2H), 6.17 (d, 1H), 7.37 (d, 2H), 7.63 (m, 3H).

### III. Examples of galenic administration forms

### A) Tablets

Tablets of the following composition are pressed on a tablet press in the customary manner:

| | |
|---|---|
| 40 mg | of substance from Example 1 |
| 120 mg | of corn starch |
| 13.5 mg | of gelatin |
| 45 mg | of lactose |
| 2.25 mg | of Aerosil® (chemically pure silicic acid in submicroscopically fine dispersion) |
| 6.75 mg | of potato starch (as a 6% paste) |

### B) Sugar-coated tablets

20 mg of substance from Example 1
60 mg of core composition
70 mg of saccharification composition

The core composition consists of 9 parts of corn starch, 3 parts of lactose and 1 part of 60:40 vinylpyrrolidone/vinyl acetate copolymer. The saccharification composition consists of 5 parts of cane sugar, 2 parts of corn starch, 2 parts of calcium carbonate and 1 part of talc. The sugar-coated tablets which had been prepared in this way are subsequently provided with a gastric juice-resistant coating.

### IV. Biological investigations

Receptor binding studies:
The substance to be tested was either dissolved in methanol/Chremophor® (BASF-AG) or in dimethyl sulfoxide and then diluted with water to the desired concentration.

### a) Dopamine D₃ receptor:

The assay mixture (0.250 ml) was composed of membranes derived from ∼ 10⁶ HEK-293 cells possessing stably expressed human dopamine D₃ receptors, 0.1 nM [¹²⁵I]-iodosulpride and incubation buffer (total binding) or, in addition, test substance (inhibition curve) or 1µM spiperone (nonspecific binding). Each assay mixture was run in triplicate.

The incubation buffer contained 50 mM tris, 120 mM NaCl, 5 mM KCI, 2 mM CaCl₂, 2 mM MgCl₂ and 0.1% bovine serum albumin, 10 µM quinolone and 0.1% ascorbic acid (prepared fresh daily). The buffer was adjusted to pH 7.4 with HCl.

### b) Dopamine D_{2L} receptor:

The assay mixture (1 ml) was composed of membranes from - 10⁶ HEK-293 cells possessing stably expressed human dopamine D_{2L} receptors (long isoform) and 0.01 nM [¹²⁵I] iodospiperone and incubation buffer (total binding) or, in addition, test substance (inhibition curve) or 1µM haloperidol (nonspecific binding). Each assay mixture was run in triplicate.

The incubation buffer contained 50 mM tris, 120 mM NaCl, 5 mM KCI, 2 mM CaCl₂, 2 mM MgCl₂ and 0.1% bovine serum albumin. The buffer was adjusted to pH 7.4 with HCl.

### c) Measurement and analysis:

After having been incubated at 25°C for 60 minutes, the assay mixtures were filtered through a Whatman GF/B glass fiber filter under vacuum using a cell collecting device. The filters were transferred to scintillation viols using a filter transfer system. After 4 ml of Ultima Gold^{®} (Packard) have been added, the samples were shaken for one hour and the radioactivity was then counted in a Beta-Counter (Packard, Tricarb 2000 or 2200CA). The cpm values were converted into dpm using a standard quench series and the program belonging to the instrument.

The inhibition curves were analyzed by means of iterative nonlinear regression analysis using the Statistical Analysis System (SAS) which is similar to the " LIGAND" program described by Munson and Rodbard.

In these tests, the compounds according to the invention exhibit very good affinities for the D₃ receptor (< 100 nM, frequently < 50 nM, in particular < 10 nM) and bind selectively to the D₃ receptor.

The results of the binding tests are given in Table 1.

+++ < 10nM, ++ <100nM, + < 1000nM

**Table 1:**

| Example | Ki (D₃) [nM] | Selectivity vs. D₂L* |
|---|---|---|
| 1 | +++ | >50 |
| 2 | +++ | >50 |
| 5 | +++ | >50 |
| 6 | +++ | >50 |
| 7 comparative | + | >10 |
| 8 | +++ | >50 |
| 9 | +++ | >50 |
| 10 | +++ | >50 |
| 11 | +++ | >50 |
| 12 | +++ | >50 |
| 13 | +++ | >50 |
| 14 | +++ | >50 |
| 15 | +++ | >50 |
| 16 | +++ | >50 |
| 17 | +++ | >50 |
| 18 | +++ | >50 |

| | | |
|---|---|---|
| * Ki(D_{2L})/Ki(D₃) | | |

## Claims

1. A N-(6-piperazin-1-ylpyridin-3-yl)benzenesulfonamide compound of the formule I 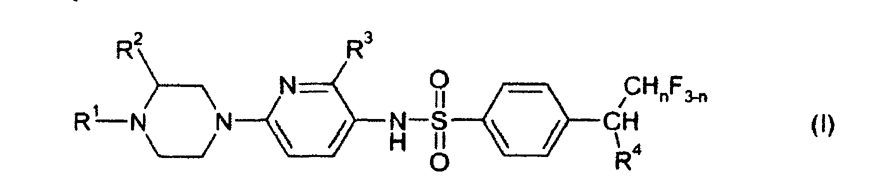 wherein
R¹ is selected from the group consisting of hydrogen, linear C₁-C₃ alkyl and
fluorinated linear C₁-C₃ alkyl;
R² is hydrogen or methyl;
R³ is selected from the group consisting of hydrogen, halogen, C₁-C₂-alkyl,
fluorinated C₁-C₂-alkyl, C₁-C₂-alkoxy and fluorinated C₁-C₂-alkoxy,
R⁴ is C₁-C₂-alkyl or fluorinated C₁-C₂-alkyl;
n is 0, 1 or 2;
and the physiologically tolerated salts of these compounds and the N-oxides thereof.

2. The compound as claimed in claim 1, wherein R¹ is hydrogen.

3. The compound as claimed in claims 1 or 2, wherein R² is methyl.

4. The compound as claimed in claim 3, wherein the carbon atom that carries R² has S-configuration.

5. The compound as claimed in claim 3, wherein the carbon atom that carries R² has R-configuration.

6. The compound as claimed in claims 1 or 2, wherein R² is hydrogen.

7. The compound as claimed in any of the preceding claims, wherein R³ is methyl.

8. The compound as claimed in any of the preceding claims, wherein R⁴ is methyl.

9. The compound as claimed in claim 8, wherein the carbon atom that carries R⁴ has S-configuration.

10. The compound as claimed in claim 8, wherein the carbon atom that carries R⁴ has R-configuration.

11. The compound as claimed in any of the preceding claims, wherein n is 1.

12. The compound as claimed in any of the preceding claims, wherein n is 2.

13. The compound according to claim 1, selected from the group consisting of:
4-(2-Fluoro-1-methyl-ethyl)-N-[2-methyl-6-(3-methyl-piperazin-1-yl)-pyridin-3-yl]-benzenesulfonamide
4-((R)-2-Fluoro-1-methyl-ethyl)-N-[2-methyl-6-((R)-3-methyl-piperazin-1-yl)-pyridin-3-yl]-benzenesulfonamide
4-((S)-2-Fluoro-1-methyl-ethyl)-N-[2-methyl-6-((S)-3-methyl-piperazin-1-yl)-pyridin-3-yl]-benzenesulfonamide.
4-((R)-2-Fluoro-1-methyl-ethyl)-N-[2-methyl-6-((S)-3-methyl-piperazin-1-yl)-pyridin-3-yl]-benzenesulfonamide
4-((S)-2-Fluoro-1-methyl-ethyl)-N-[2-methyl-6-((R)-3-methyl-piperazin-1-yl)-pyridin-3-yl]-benzenesulfonamide
4-(2-Fluoro-1-methyl-ethyl)-N-[2-methyl-6-piperazin-1-yl-pyridin-3-yl]-benzenesulfonamide
4-((R)-2-Fluoro-1-methyl-ethyl)-N-[2-methyl-6-piperazin-1-yl-pyridin-3-yl]-benzenesulfonamide
4-((S)-2-Fluoro-1-methyl-ethyl)-N-[2-methyl-6-piperazin-1-yl-pyridin-3-yl]-benzenesulfonamide
4-(2,2-Difluoro-1-methyl-ethyl)-N-(2-methyl-6-piperazin-1-yl-pyridin-3-yl)-benzenesulfonamide
4-((S)-2,2-Difluoro-1-methyl-ethyl)-N-(2-methyl-6-piperazin-1-yl-pyridin-3-yl)-benzenesulfonamide
4-((R)-2,2-Difluoro-1-methyl-ethyl)-N-(2-methyl-6-piperazin-1-yl-pyridin-3-yl)-benzenesulfonamide
4-(2,2,2-Trifluoro-1-methyl-ethyl)-N-(2-methyl-6-piperazin-1-yl-pyridin-3-yl)-benzenesulfonamide
4-((R)-2,2,2-Trifluoro-1-methyl-ethyl)-N-(2-methyl-6-piperazin-1-yl-pyridin-3-yl)-benzenesulfonamide
4-((S)-2,2,2-Trifluoro-1-methyl-ethyl)-N-(2-methyl-6-piperazin-1-yl-pyridin-3-yl)-benzenesulfonamide
4-(2-Fluoro-1-methyl-ethyl)-N-(2-methoxy-6-piperazin-1-yl-pyridin-3-yl)-benzenesulfonamide
4-((R)-2-Fluoro-1-methyl-ethyl)-N-(2-methoxy-6-piperazin-1-ylpyridin-3-yl)-benzenesulfonamide
4-((S)-2-Fluoro-1-methyl-ethyl)-N-(2-methoxy-1-piperazin-1-yl-pyridin-3-yl)-benzenesulfonamide
4-(2,2-Difluoro-1-methyl-ethyl)-N-(2-methoxy-6-piperazin-1-yl-pyridin-3-yl)-benzenesulfonamide
4-((S)-2,2-Difluoro-1-methyl-ethyl)-N-(2-methoxy-6-piperazin-1-yl-pyridin-3-yl)-benzenesulfonamide
4-((R)-2,2-Difluoro-1-methyl-ethyl)-N-(2-methoxy-6-piperazin-1-yl-pyridin-3-yl)-benzenesulfonamide
N-(2-Methoxy-6-piperazin-1-yl-pyridin-3-yl)-4-(2,2,2-trifluoro-1-methyl-ethyl)-benzenesulfonamide
N-(2-Methoxy-6-piperazin-1-yl-pyridin-3-yl)-4-((S)-2,2,2-triftuoro-1-methyl-ethyl)-benzenesulfonamide
N-(2-Methoxy-6-piperazin-1-yl-pyridin-3-yl)-4-((R)-2,2,2-trifluoro-1-methyl-ethyl)-benzenesulfonamide
and the physiologically tolerated salts of these compounds.

14. A pharmaceutical composition comprising at least one compound as claimed in any of claims 1 to 13, optionally together with at least one physiologically acceptable carrier or auxiliary substance.

15. A compound as claimed in any of claims 1 to 13 for the use as a medicament.

16. The compound as claimed in any of claims 1 to 13 for the use as a medicament for treating a medical disorder susceptible to treatment with a dopamine D₃ receptor ligand.

17. The compound as claimed in any of claims 1 to 13, for use as a medicament for treating a medical disorder, wherein the medial disorder is selected from schizophrenia, drug addiction, diabetic nephropathy and bipolar disorder.

18. A compound of the formula IV 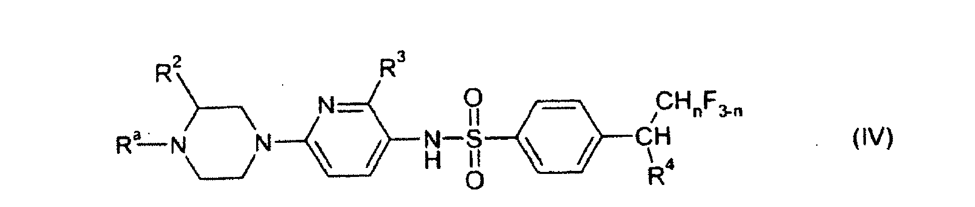 wherein R^{a} is an N-protecting group and wherein n, R², R³ and R⁴ are as defined in claims 1 to 12.

## Patentansprüche

1. N-(6-Piperazin-1-ylpyridin-3-yl)benzensulfonamid-Verbindung der Formel I 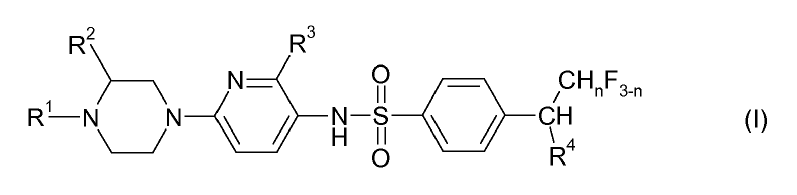 worin
R¹ ausgewählt ist unter Wasserstoff, linearem C₁-C₃-Alkyl und fluoriertem linearen C₁-C₃-Alkyl;
R² für Wasserstoff oder Methyl steht;
R³ ausgewählt ist unter Wasserstoff, Halogen, C₁-C₂-Alkyl, fluoriertem C₁-C₂-Alkyl, C₁-C₂-Alkoxy und fluoriertem C₁-C₂-Alkoxy;
R⁴ für C₁-C₂-Alkyl oder fluoriertes C₁-C₂-Alkyl steht;
n für 0, 1 oder 2 steht;
sowie die physiologisch verträglichen Salze dieser Verbindungen und deren N-Oxide.

2. Verbindung nach Anspruch 1, worin R¹ für Wasserstoff steht.

3. Verbindung nach Anspruch 1 oder 2, worin R² für Methyl steht.

4. Verbindung nach Anspruch 3, worin das R² tragende Kohlenstoffatom S-Konfiguration aufweist.

5. Verbindung nach Anspruch 3, worin das R² tragende Kohlenstoffatom R-Konfiguration aufweist.

6. Verbindung nach Anspruch 1 oder 2, worin R² für Wasserstoff steht.

7. Verbindung nach einem der vorhergehenden Ansprüche, worin R³ für Methyl steht.

8. Verbindung nach einem der vorhergehenden Ansprüche, worin R⁴ für Methyl steht.

9. Verbindung nach Anspruch 8, worin das R⁴ tragende Kohlenstoffatom S-Konfiguration aufweist.

10. Verbindung nach Anspruch 8, worin das R⁴ tragende Kohlenstoffatom R-Konfiguration aufweist.

11. Verbindung nach einem der vorhergehenden Ansprüche, worin n für 1 steht.

12. Verbindung nach einem der vorhergehenden Ansprüche, worin n für 2 steht.

13. Verbindung nach Anspruch 1, ausgewählt unter:
4-(2-Fluor-1-methyl-ethyl)-N-[2-methyl-6-(3-methyl-piperazin-1-yl)-pyrid in-3-yl]-benzensulfonamid
4-((R)-2-Fluor-1-methyl-ethyl)-N-[2-methyl-6-((R)-3-methyl-piperazin-1-yl)-pyridin-3-yl]-benzensulfonamid
4-((S)-2-Fluor-1-methyl-ethyl)-N-[2-methyl-6-((S)-3-methyl-piperazin-1-yl)-pyridin-3-yl]-benzensulfonamid
4-((R)-2-Fluor-1-methyl-ethyl)-N-[2-methyl-6-((S)-3-methyl-piperazin-1-yl)-pyridin-3-yl]-benzensulfonamid
4-((S)-2-Fluor-1-methyl-ethyl)-N-[2-methyl-6-((R)-3-methyl-piperazin-1-yl)-pyridin-3-yl]-benzensulfonamid
4-(2-Fluor-1-methyl-ethyl)-N-[2-methyl-6-piperazin-1-yl-pyridin-3-yl]-benzensulfonamid
4-((R)-2-Fluor-1-methyl-ethyl)-N-[2-methyl-6-piperazin-1-yl-pyridin-3-yl]-benzensulfonamid
4-((S)-2-Fluor-1-methyl-ethyl)-N-[2-methyl-6-piperazin-1-yl-pyridin-3-yl]-benzensulfonamid
4-(2,2-Difluor-1-methyl-ethyl)-N-(2-methyl-6-piperazin-1-yl-pyridin-3-yl)-benzensulfonamid
4-((S)-2,2-Difluor-1-methyl-ethyl)-N-(2-methyl-6-piperazin-1-yl-pyridin-3-yl)-benzensulfonamid
4-((R)-2,2-Difluor-1-methyl-ethyl)-N-(2-methyl-6-piperazin-1-yl-pyridin-3-yl)-benzensulfonamid
4-(2,2,2-Trifluor-1-methyl-ethyl)-N-(2-methyl-6-piperazin-1-yl-pyridin-3-yl)-benzensulfonamid
4-((R)-2,2,2-Trifluor-1-methyl-ethyl)-N-(2-methyl-6-piperazin-1-yl-pyridin-3-yl)-benzensulfonamid
4-((S)-2,2,2-Trifluor-1-methyl-ethyl)-N-(2-methyl-6-piperazin-1-yl-pyridin-3-yl)-benzensulfonamid
4-(2-Fluor-1-methyl-ethyl)-N-(2-methoxy-6-piperazin-1-yl-pyrid in-3-yl)-benzensulfonamid
4-((R)-2-Fluor-1-methyl-ethyl)-N-(2-methoxy-6-piperazin-1-yl-pyridin-3-yl)-benzensulfonamid
4-((S)-2-Fluor-1-methyl-ethyl)-N-(2-methoxy-6-piperazin-1-yl-pyridin-3-yl)-benzensulfonamid
4-(2,2-Difluor-1-methyl-ethyl)-N-(2-methoxy-6-piperazin-1-yl-pyridin-3-yl)-benzensulfonamid
4-((S)-2,2-Difluor-1-methyl-ethyl)-N-(2-methoxy-6-piperazin-1-yl-pyridin-3-yl)-benzensulfonamid
4-((R)-2,2-Difluor-1-methyl-ethyl)-N-(2-methoxy-6-piperazin-1-yl-pyridin-3-yl)-benzensulfonamid
N-(2-Methoxy-6-piperazin-1-yl-pyridin-3-yl)-4-(2,2,2-trifluor-1-methyl-ethyl)-benzensulfonamid
N-(2-Methoxy-6-piperazin-1-yl-pyridin-3-yl)-4-((S)-2,2,2-trifluor-1-methyl-ethyl)-benzensulfonamid
N-(2-Methoxy-6-piperazin-1-yl-pyridin-3-yl)-4-((R)-2,2,2-trifluor-1-methyl-ethyl)-benzensulfonamid
und die physiologisch verträglichen Salze dieser Verbindungen.

14. Pharmazeutisches Mittel, enthaltend wenigstens eine Verbindung nach einem der Ansprüche 1 bis 13, gegebenenfalls zusammen mit wenigstens einem physiologisch akzeptablen Träger oder Hilfsstoff.

15. Verbindung nach einem der Ansprüche 1 bis 13 zur Verwendung als Medikament.

16. Verbindung nach einem der Ansprüche 1 bis 13 zur Verwendung als Medikament zur Behandlung einer Erkrankung, die auf die Beeinflussung durch einen Dopamin-D₃-Rezeptorliganden anspricht.

17. Verbindung nach einem der Ansprüche 1 bis 13 zur Verwendung als Medikament zur Behandlung einer Erkrankung, wobei die Erkrankung ausgewählt ist unter Schizophrenie, Drogenabhängigkeit, diabetischer Nephropathie und bipolarer Störung.

18. Verbindung der Formel IV 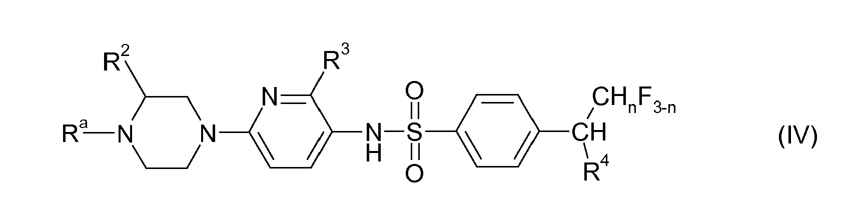 worin R^{a} für eine N-Schutzgruppe steht and worin n, R², R³ und R⁴ wie in einem der Ansprüche 1 bis 12 definiert sind.

## Revendications

1. Composé de
N-(6-pipérazin-1-yl-pyridin-3-yl)benzènesulfonamide de formule I 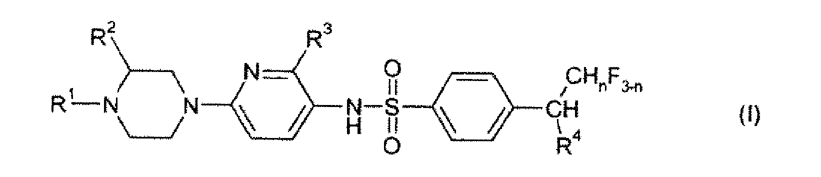 dans laquelle
R¹ est choisi dans le groupe constitué d'hydrogène, C₁-C₃₋alkyle linéaire et C₁-C₃₋alkyle linéaire fluoré ;
R² est hydrogène ou méthyle ;
R³ est choisi dans le groupe constitué d'hydrogène, halogène, C₁-C₂-alkyle, C₁-C₂-alkyle fluoré, C₁-C₂₋alcoxy, et C₁-C₂₋alcoxy fluoré ;
R⁴ est C₁-C₂₋alkyle ou C₁-C₂₋alkyle fluoré ;
n est 0, 1 ou 2 ;
et les sels physiologiquement tolérés de ces composés et les N-oxydes de ceux-ci.

2. Composé selon la revendication 1, **caractérisé en ce que** R¹ est hydrogène.

3. Composé selon la revendication 1 ou 2, **caractérisé en ce que** R² est méthyle.

4. Composé selon la revendication 3, **caractérisé en ce que** l'atome de carbone qui porte R² a la configuration S.

5. Composé selon la revendication 3, **caractérisé en ce que** l'atome de carbone qui porte R² a la configuration R.

6. Composé selon la revendication 1 ou 2, **caractérisé en ce que** R² est hydrogène.

7. Composé selon l'une quelconque des revendications précédentes, **caractérisé en ce que** R³ est méthyle.

8. Composé selon l'une quelconque des revendications précédentes, **caractérisé en ce que** R⁴ est méthyle.

9. Composé selon la revendication 8, **caractérisé en ce que** l'atome de carbone qui porte R⁴ a la configuration S.

10. Composé selon la revendication 8, **caractérisé en ce que** l'atome de carbone qui porte R⁴ a la configuration R.

11. Composé selon l'une quelconque des revendications précédentes, **caractérisé en ce que** n est 1.

12. Composé selon l'une quelconque des revendications précédentes, **caractérisé en ce que** n est 2.

13. Composé selon la revendication 1, choisi dans le groupe constitué de :
4-(2-fluoro-1-méthyl-éthyl)-N-[2-méthyl-6-(3-méthyl-pipérazin-1-yl)-pyridin-3-yl]-benzènesulfonamide
4-((R)-2-fluoro-1-méthyl-éthyl)-N-[2-méthyl-6-((R)-3-méthyl-pipérazin-1-yl)-pyridin-3-yl]-benzènesulfonamide
4-((S)-2-fluoro-1-méthyl-éthyl)-N-[2-méthyl-6-((S)-3-méthyl-pipérazin-1-yl)-pyridin-3-yl]-benzènesulfonamide
4-((R)-2-fluoro-1-méthyl-éthyl)-N-[2-méthyl-6-((S)-3-méthyl-pipérazin-1-yl)-pyridin-3-yl]-benzènesulfonamide
4-((S)-2-fluoro-1-méthyl-éthyl)-N-(2-méthyl-6-((R)-3-méthyl-pipérazin-1-yl)-pyridin-3-yl]-benzènesulfonamide
4-(2-fluoro-1-méthyl-éthyl)-N-[2-méthyl-6-pipérazin-1 -yl-pyridin-3-yl]-benzènesulfonamide
4-((R)-2-fluoro-1-méthyl-éthyl)-N-[2-méthyl-6-pipérazin-1-yl-pyridin-3-yl]-benzènesulfonamide
4-((S)-2-fluoro-1-méthyl-éthyl)-N-[2-méthyl-6-pipérazin-1-yl-pyridin-3-yl]-benzènesulfonamide
4-(2,2-difluoro-1-méthyl-éthyl)-N-(2-méthyl-6-pipérazin-1-yl-pyridin-3-yl)-benzènesulfonamide
4-((S)-2,2-difluoro-1-méthyl-éthyl)-N-(2-méthyl-6-pipérazin-1-yl-pyridin-3-yl)-benzènesulfonamide
4-((R)-2,2-difluoro-1-méthyl-éthyl)-N-(2-méthyl-6-pipérazin-1-yl-pyridin-3-yl)-benzènesulfonamide
4-(2,2,2-trifluoro-1-méthyl-éthyl)-N-(2-méthyl-6-pipérazin-1-yl-pyridin-3-yl)-benzènesulfonamide
4-((R)-2,2,2-trifluoro-1-méthyl-éthyl)-N-(2-méthyl-6-pipérazin-1-yl-pyridin-3-yl)-benzènesulfonamide
4-((S)-2,2,2-trifluoro-1-méthyl-éthyl)-N-(2-méthyl-6-pipérazin-1-yl-pyridin-3-yl)-benzènesulfonamide 4-(2-fluoro-1-méthyl-éthyl)-N-(2-méthoxy-6-pipérazin-1-yl-pyridin-3-yl)-benzènesulfonamide
4-((R)-2-fluoro-1-méthyl-éthyl)-N-(2-méthoxy-6-pipérazin-1-yl-pyridin-3-yl)-benzènesulfonamide
4-((S)-2-fluoro-1-méthyl-éthyl)-N-(2-méthoxy-6-pipérazin-1-yl-pyridin-3-yl)-benzènesulfonamide
4-(2,2-difluoro-1-méthyl-éthyl)-N-(2-méthoxy-6-pipérazin-1-yl-pyridin-3-yl)-benzènesulfonamide
4-((S)-2,2-difluoro-1-méthyl-éthyl)-N-(2-méthoxy-6-pipérazin-1-yl-pyridin-3-yl)-benzènesulfonamide
4-((R)-2,2-difluoro-1-méthyl-éthyl)-N-(2-méthoxy-6-pipérazin-1-yl-pyridin-3-yl)-benzènesulfonamide
N-(2-méthoxy-6-pipérazin-1-yl-pyridin-3-yl)-4-(2,2,2-trifluoro-1-méthyl-éthyl)-benzènesulfonamide
N-(2-méthoxy-6-pipérazin-1-yl-pyridin-3-yl)-4-((S)-2, 2,2-trifluoro-1-méthyl-éthyl)-benzènesulfonamide
N-(2-méthoxy-6-pipérazin-1-yl-pyridin-3-yl)-4-((R)-2, 2,2-trifluoro-1-méthyl-éthyl)-benzènesulfonamide
et les sels physiologiquement tolérés de ces composés.

14. Composition pharmaceutique comprenant au moins un composé selon l'une quelconque des revendications 1 à 13, éventuellement conjointement avec au moins un support ou une substance auxiliaire physiologiquement acceptable.

15. Composé selon l'une quelconque des revendications 1 à 13, destiné à être utilisé comme médicament.

16. Composé selon l'une quelconque des revendications 1 à 13, destiné à être utilisé comme médicament pour traiter un trouble médical sensible aux traitements par un ligand du récepteur D₃ de la dopamine.

17. Composé selon l'une quelconque des revendications 1 à 13, destiné à être utilisé comme médicament pour traiter un trouble médical, le trouble médical étant choisi parmi la schizophrénie, la toxicomanie, la névropathie diabétique et le trouble bipolaire.

18. Composé de formule IV 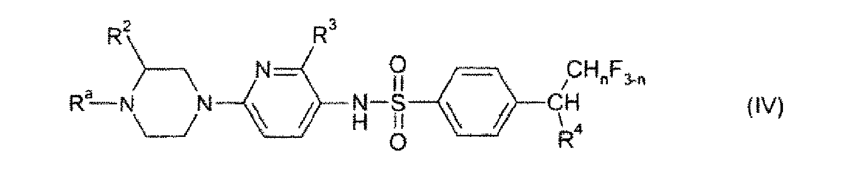 dans laquelle R^{a} est un groupement protecteur de N et dans laquelle n, R², R³ et R⁴ sont tels que définis dans les revendications 1 à 12.
